# EUROPEAN PATENT APPLICATION

(11) **EP 4 717 259 A1**
(43) Date of publication of application: **01.04.2026**
(21) Application number: 25201605.0
(22) Date of filing: 11.09.2025
(51) Int. Cl.: A61K 9/16, A61K 9/28, A61K 9/50, A61K 31/00

(54) **PHARMACEUTICAL COMPOSITION COMPRISING ROSUVASTATIN AND ACETYLSALICYLIC ACID, PROCESS FOR PREPARING THE SAME, AND USES THEREOF**

(30) Priority: 12.09.2024 MX 2024011227
(71) Applicant: Laboratorios Silanes, S.A. de C.V., Mexico, 11000 (MX)
(72) Inventor: OLLERVIDES RUBIO, Paola Yazmín, 11000 Ciudad de México (MX); FARFÁN SALAZAR, Claudia Delfina, 11000 Ciudad de México (MX); GONZÁLEZ CANUDAS, Jorge Alejandro, 11000 Ciudad de México (MX); RIOS BRITO, Kevin Francisco, 11000 Ciudad de México (MX)
(74) Representative: Hoffmann Eitle

(57) **Abstract**

The present invention provides a pharmaceutical composition comprising a) a first phase comprising rosuvastatin, and b) a second phase comprising acetylsalicylic acid, wherein the first phase comprising rosuvastatin is in the form of a coated tablet, and the second phase comprising acetylsalicylic acid is in the form of granules; said pharmaceutical composition solves a set of significant technological challenges due to the physicochemical properties and difference in dosage of each drug. The pharmaceutical composition of the present invention is stable, and also has a comparative bioavailability equivalent to that of the co-administered monodrugs, so that the composition is safe and effective in the treatment of hyperlipidemias and in the prevention of at least one cardiovascular disease. The invention is also directed to a process for manufacturing the pharmaceutical composition.

## Description

### TECHNICAL FIELD

The present invention relates to the field of the pharmaceutical industry and more particularly to a pharmaceutical composition comprising rosuvastatin and acetylsalicylic acid, a process for preparing the same, and uses thereof.

### BACKGROUND

Cardiovascular events are one of the leading causes of morbidity and mortality worldwide, constituting a significant public health challenge. In recent decades, there has been an alarming increase in the incidence of such events within the population, underscoring the urgency of implementing effective prevention and treatment strategies.

The use of medicaments, such as statins to control cholesterol, antihypertensives to regulate blood pressure, and antiplatelet agents to prevent blood clots plays a key role in reducing cardiovascular risk.

In this regard, comparative studies between various doses of rosuvastatin and various doses of other statins, such as atorvastatin, pravastatin, and simvastatin, have been published mainly in terms of their performance in reducing cholesterol and adverse events. Rosuvastatin performed better in reducing cholesterol, even at lower doses than atorvastatin and the other statins indicated.

Rosuvastatin is a fully synthetic drug, a member of the statin family, and a lipid-lowering agent whose chemical name is (bis[(E)-7-[4-(4-fluorophenyl)-6-isopropyl-2-[methyl(methylsulfonyl)amino]pyrimidin-5-yl] (3R,5S)-3,5-dihydroxyhept-6-enoic acid]. Rosuvastatin is a selective, competitive HMG-CoA reductase inhibitor, a rate-limiting enzyme that converts 3-hydroxy-3-methylglutaryl-coenzyme A to mevalonate, a precursor of cholesterol.

Acetylsalicylic acid (ASA), commonly known as aspirin, is a non-steroidal anti-inflammatory drug (NSAID) belonging to the salicylate family; its chemical name is 2-(acetyloxy)benzoic acid; salicylic acid acetate. ASA interferes with the production of prostaglandins in various organs and tissues through acetylation of the enzyme cyclo-oxygenase. By preventing the synthesis and release of prostaglandins in inflammatory processes, ASA may prevent the sensitization of pain receptors.

Considering the mechanisms of action and effects on the human body, formulations comprising a combination of the active ingredients rosuvastatin and acetylsalicylic acid have been developed for the secondary prevention of cardiovascular events. However, it is known that acetylsalicylic acid has acidic properties, while rosuvastatin incorporates alkaline salts, so that the mixture of acetylsalicylic acid and rosuvastatin can result in the hydrolysis of acetylsalicylic acid, leading to rosuvastatin degradation. Rosuvastatin salts are particularly unstable and susceptible to heat, humidity, low pH, and light. Acetylsalicylic acid, on the other hand, is an ester that is easily hydrolyzed in the presence of moisture to form salicylic acid. Furthermore, according to its biopharmaceutics classification Class II, rosuvastatin has high permeability and low solubility, which jeopardizes its stability, and consequently, the pharmaceutical composition or pharmaceutical form containing it. In order to try to solve the above problems, pharmaceutical compositions have been proposed to combine these active ingredients in separate entities into a single solid dosage form, whether tablets, particles, granules, pellets, or capsules.

U.S. Patent Application Publication No. 2007/116756 A1, published on May 24, 2007 (DR. REDDY LAB. INC.), discloses stable pharmaceutical compositions of cardiovascular therapeutic agents, comprising a combination of effective doses of a cholesterol-lowering agent, or its pharmaceutically acceptable salts, solvates, enantiomers or mixtures thereof in a single dosage form, and aspirin. The amount of aspirin ranges from 25 to 600 mg. Statins are among the useful cholesterol-lowering agents, which include, but are not limited to, atorvastatin, pravastatin, cerivistatin, fluindostatin, fluvastatin, lovastatin, mevastatin, rosuvastatin, pitvastatin, dalvastatin and velostatin. Although the application discloses that said compositions are stable, stability was only tested for a composition comprising lisinopril, atenolol, and aspirin for 4 weeks. In contrast, in the present invention a composition comprising rosuvastatin and acetylsalicylic acid was stability tested for 6 months.

International Application Publication No. WO 2012/081905, published on June 21, 2012 (HANMI HOLDING, CO), discloses a composite formulation for preventing or treating cardiovascular diseases, which comprises a first particle comprising an HMG-CoA reductase inhibitor and a basic additive, and a second particle comprising an aspirin core and an enteric coating layer, wherein the difference in the average diameters of said first and second particles is 100 µm to 800 µm. Examples of HMG-CoA reductase inhibitors include rosuvastatin, lovastatin, atorvastatin, pravastatin, fluvastatin, pitavastatin, simvastatin, rivastatin, cerivastatin, velostatin, mevastatin, and pharmaceutically acceptable salts, precursors thereof, or a mixture thereof. The first particle may further comprise diluents and other excipients such as disintegrants, binders, lubricants, coating agents, fillers, rheology modifiers, crystallization retarders, solubilizers, pH modifiers, surfactants, emulsifiers, or mixtures thereof. The second particle comprises an aspirin core and an enteric coating that may include excipients such as plasticizers and lubricants. The present invention provides a pharmaceutical composition comprising a first rosuvastatin phase and a second stable acetylsalicylic acid phase, wherein only one of the phases is in the form of granules.

Further, International Application Publication No. WO 2012/011882 A1, published on January 26, 2012 (MAHMUT BILGIC), discloses a pharmaceutical composition comprising aspirin (acetylsalicylic acid) and atorvastatin and/or their pharmaceutically acceptable salts, hydrates, enantiomers, racemates, organic salts, inorganic salts, esters, polymorphs, crystal forms, amorphous forms, and combinations thereof. The active ingredients are formulated into separate units being presented in a single dosage form. Said units can be in the form of granules, powder, pellet, tablet, mini tablet, micro tablet, capsule, micro capsule, and combinations thereof. It was found that the highest bioavailability is achieved in tablet and powder forms, preferably atorvastatin powder and aspirin tablet. In contrast, the present invention provides a pharmaceutical composition comprising a first rosuvastatin phase and a second acetylsalicylic acid phase, wherein preferably the first rosuvastatin phase is in the form of a tablet and the second acetylsalicylic acid phase is in the form of granules.

Likewise, U.S. Patent Application Publication No. 2002/034546 A1, published on March 21, 2002 (ISMAT ULLAH & NEMICHAND B JAIN), discloses a composition comprising a statin cholesterol lowering agent and aspirin. In some embodiments, each of the drugs is in separate units disposed within a dosage unit. The separate forms include granules and excipients, commonly known in the art. However, statins do not include rosuvastatin; and in certain embodiments, aspirin is present as coated granules.

International Application Publication No. WO 2014/195421 A1, published on December 11, 2014 (FERRER INT., SA & FUNDACIÓN CENTRO NACIONAL DE INVESTIGACIONES CARDIOVASCULARES CARLOS III), discloses an orally administrable pharmaceutical dosage form for the treatment and/or prevention of cardiovascular diseases comprising acetylsalicylic acid and an HMG-CoA reductase inhibitor selected from atorvastatin and rosuvastatin and salts thereof, said active ingredients being in two or more separate coated dosage units comprising one or more water-soluble polymers in said coating, being free of water-insoluble polymers or an enteric polymer, wherein the coating is above 6 mg/cm² and does not show a modified release profile; wherein said separate coated dosage units are selected from the group consisting of tablets, particles, granules, pellets, and capsules. The pharmaceutical composition of the present invention comprises a first rosuvastatin phase and a second acetylsalicylic acid phase; however, only one of the phases is coated.

U.S. Patent Application Publication No. US2012/045505 A1, published on February 23, 2012 (DR. REDDY LAB. INC.), discloses a pharmaceutical formulation in the form of a capsule containing at least one tablet, comprising as active ingredients: a cholesterol-lowering agent, aspirin, an inhibitor of the renin-angiotensin system, and a beta-adrenergic receptor blocking agent or a diuretic, the active ingredients or combination of two or more thereof, being present in the form of tablets, capsules, or particles, wherein the cholesterol-lowering agent is physically separated from aspirin. The cholesterol-lowering agent may be lovastatin, atorvastatin, simvastatin, fluvastatin, rosuvastatin, or pravastatin, their salts, esters, amides, prodrugs, metabolites, analogues, solvates, hydrates, enantiomers, polymorphs, derivatives, and the like. According to the examples in this document, aspirin is present in the form of a coated tablet; in contrast, the pharmaceutical composition of the present invention comprises a second acetylsalicylic acid phase in the form of granules.

Document No. WO 2012/124973 A2, published on September 20, 2012 (BORYUNG PHARM), discloses a capsule dosage form of a combined drug formulation for the treatment of cardiovascular diseases, which comprises mini-tablets containing a cholesterol-lowering agent, a stabilizer thereof, and excipients, including an enteric coating on a surface thereof, having a diameter of 7.5 mm or less; and antithrombotic agent mini-tablets or mini-pellets and excipients, including an enteric coating on a surface thereof, having a diameter of 7.5 mm or less. The cholesterol-lowering agent may be rosuvastatin, rosuvastatin calcium, atorvastatin calcium, rivastatin, and pharmaceutically acceptable salts thereof. The stabilizer may be calcium phosphate. The preferred antithrombotic agent is acetylsalicylic acid. Similarly to the above documents, it discloses that acetylsalicylic acid has an enteric coating, which may alter the drug release; in contrast, the pharmaceutical composition of the present invention comprises a non-coated acetylsalicylic acid phase.

International Publication No. WO 2004/080488 A1, published on September 23, 2004 (BAYER HEALTHCARE AG), generally discloses a combination of acetylsalicylic acid and a statin, in separate forms (tablets or capsules) one of them being within a packaging material (blister pack). Said document does not teach or suggest that the drugs can be in a single pharmaceutical form.

Also, document No. WO 2011/096665 A2, published on August 11, 2011 (HANMI HOLDING, CO), discloses a capsule formulation containing aspirin granules or pellets coated with a barrier containing a hydrophobic additive, and granules or pellets of an HMG-CoA reductase inhibitor. Aspirin is in an amount of from 10 mg to 2 g. The HMG-CoA reductase inhibitor may be mevastatin, rosuvastatin, atorvastatin, lovastatin, pravastatin, pravastatin lactone, pitavastatin, bervastatin, velostatin, simvastatin, rivastatin, fluvastatin, cerivastatin or isomers or salts and combinations thereof. The HMG-CoA reductase inhibitor is in an amount of from 5 mg to 80 mg. Unlike this document, the pharmaceutical composition of the present invention comprises acetylsalicylic acid (aspirin) without an enteric coating.

Document CN 1965843 A, published on May 23, 2007 (BEIJING HUAANFO MEDICAL RES CT), discloses the use of a combination of aspirin and a statin cholesterol-lowering agent (simvastatin, pravastatin, lovastatin, fluvastatin, atorvastatin, rosuvastatin, pitavastatin) in preparing a medicament for the treatment of hyperviscosemia. Aspirin is in the form of coated tablets, coated micro-pills, or coated drop-shaped pills, wherein the coating is an enteric coating. As indicated above, the pharmaceutical composition of the present invention comprises an acetylsalicylic acid phase that has no enteric coating.

None of the prior art documents propose improving the stability of a pharmaceutical composition comprising a statin and acetylsalicylic acid, said NSAID being formulated without a coating, despite the well-known problems of statin susceptibility to heat, humidity, low pH environments, and light; or to drug interactions that can lead to degradation products, which shorten shelf-life thereof. Furthermore, rosuvastatin is sensitive to moisture, and in typical granulation processes, such as wet granulation, there is residual moisture that jeopardizes the stability of rosuvastatin and, consequently, of any pharmaceutical form manufactured by said procedure that contains it.

As a result of the above, efforts have been made to eliminate the drawbacks related to stability of pharmaceutical compositions comprising rosuvastatin and acetylsalicylic acid as active ingredients by developing a pharmaceutical composition comprising rosuvastatin and acetylsalicylic acid, a process for preparing the same, and uses thereof, wherein a single, solid, stable, immediate-release pharmaceutical form is provided. The proposed composition and manufacturing process thereof overcome the major drawbacks associated with the combination of active ingredients through its novel manufacturing process.

Thus, the present invention proposes a combination of the active ingredients rosuvastatin and acetylsalicylic acid into a single dosage unit, thereby solving a set of significant technological challenges due to the physicochemical properties and difference in dosage to ensure obtaining a stable product for treating and preventing cardiovascular diseases.

### OBJECTS OF THE INVENTION

Considering the shortcomings of the prior art, it is an object of the present invention to provide a solid pharmaceutical composition comprising rosuvastatin and acetylsalicylic acid, wherein said pharmaceutical composition is stable, immediate release, and easy to administer.

Another object of the invention is to provide a pharmaceutical composition comprising rosuvastatin and acetylsalicylic acid whose dissolution profile is comparable to the separate monodrugs.

A further object of the invention is to provide a process for manufacturing a pharmaceutical composition comprising rosuvastatin and acetylsalicylic acid, improving the stability of the active ingredients.

These and other objects are achieved by a pharmaceutical composition comprising rosuvastatin and acetylsalicylic acid, a process for preparing the same, and uses thereof in accordance with the present invention.

### SUMMARY OF THE INVENTION

To this end, a first aspect of the present invention relates to a pharmaceutical composition comprising:
a) a first phase comprising rosuvastatin; and
b) a second phase comprising acetylsalicylic acid,
wherein the first phase comprising rosuvastatin is in the form of a coated tablet, and the second phase comprising acetylsalicylic acid is in the form of granules.

A second aspect of the invention provides a process for manufacturing the pharmaceutical composition described above, wherein the process comprises the stages of:
a) providing a first phase comprising rosuvastatin, wherein the first phase comprising rosuvastatin is in the form of a coated tablet;
b) providing a second phase comprising acetylsalicylic acid, wherein the second phase comprising acetylsalicylic acid is in the form of granules; and
c) encapsulating the first phase comprising rosuvastatin and the second phase comprising acetylsalicylic acid.

A third aspect of the invention relates to the use of a pharmaceutical composition of the present invention in treating hyperlipidemia and preventing at least one cardiovascular disease.

### BRIEF DESCRIPTION OF THE DRAWINGS

The novel aspects considered as characteristic of the present invention will be set forth particularly in the appended claims. However, some embodiments, features, and some objects and advantages thereof will be better understood from the detailed description when read in conjunction with the accompanying drawings, in which:
Figure 1 is a flowchart of a first stage of the process for preparing a pharmaceutical composition according to one embodiment of the invention.
Figure 2 shows a flowchart of a second stage and a third stage of the process for preparing a pharmaceutical composition according to one embodiment of the invention.
Figure 3 depicts a plot showing the results of an accelerated stability study relating to rosuvastatin content of a pharmaceutical composition according to one embodiment of the invention.
Figure 4 is a plot showing the results of an accelerated stability study relating to acetylsalicylic acid content of a pharmaceutical composition according to one embodiment of the invention.
Figure 5 depicts a plot showing the results of an accelerated stability study relating to the dissolution of rosuvastatin present in a pharmaceutical composition according to one embodiment of the invention.
Figure 6 depicts a plot showing the results of an accelerated stability study relating to the dissolution of acetylsalicylic acid present in a pharmaceutical composition according to one embodiment of the invention.
Figure 7 shows a diagram of a comparative bioavailability study design.
Figure 8 is a mean plasma concentration-time profile on an arithmetic scale of rosuvastatin after medicament administration.
Figure 9 depicts a mean plasma concentration-time profile on a semi-logarithmic scale of rosuvastatin after medicament administration.
Figure 10 shows a mean plasma concentration-time profile on an arithmetic scale of acetylsalicylic acid after medicament administration.
Figure 11 is a mean plasma concentration-time profile on a semi-logarithmic scale of acetylsalicylic acid after medicament administration.

### DETAILED DESCRIPTION

It should be understood that the present invention is not limited to the methodologies, protocols, and reagents provided by the detailed description, as these may vary. It should also be understood that the terminology used herein is merely for describing particular embodiments and is not intended to limit the scope of the present disclosure, which will only be limited by the accompanying claims. Unless otherwise defined, all technical and scientific terms used herein have the same meaning as those commonly understood by a person skilled in the art.

### Definitions

As used in the present invention, the term "approximately" refers to a measurable value that, due to the nature of the techniques used to measure it, may vary. In the case of an amount of weight, time, dose, etc., it is understood to encompass, for example, deviations of ± 20% or ± 10%, as another example ± 5%, as another example ± 1%, and as another example ± 0.1% from a specified amount, as such deviations are appropriate for carrying out the disclosed process.

The term "pharmaceutically acceptable salt" of a given compound refers to salts that retain the biological efficacy and properties of the given compound and are not biologically or otherwise undesirable. Pharmaceutically acceptable base addition salts can be prepared from inorganic or organic bases. Salts derived from inorganic bases include, by way of example only, sodium, potassium, lithium, ammonium, calcium, and magnesium salts.

As used herein, the term "stability" is defined as the ability of a pharmaceutical product to retain its chemical, physical, microbiological, and biopharmaceutical properties within specified limits throughout its shelf-life.

The present invention relates to solid, stable, immediate-release pharmaceutical compositions that retain dissolution and bioavailability of a statin and a non-steroidal anti-inflammatory drug (NSAID). Specifically, in the compositions of the present invention, said statin is rosuvastatin and said NSAID is acetylsalicylic acid. In this regard, it is known that the combination of rosuvastatin and acetylsalicylic acid presents a set of significant technological challenges due to drug physicochemical properties, since mixing acetylsalicylic acid and rosuvastatin can result in the degradation of both active ingredients.

In order to solve the aforementioned technical problem, the present invention provides a pharmaceutical composition comprising:
a) a first phase comprising rosuvastatin; and
b) a second phase comprising acetylsalicylic acid,
wherein the first phase comprising rosuvastatin is in the form of a coated tablet, and the second phase comprising acetylsalicylic acid is in the form of granules.

The pharmaceutical composition further comprises pharmaceutically acceptable excipients, which are selected from the group consisting of diluents, carriers, stabilizing agents, disintegrants, glidants, lubricants, coatings, binders, wetting agents, and adsorbents.

Examples of pharmaceutically acceptable diluents include, but are not limited to, lactose monohydrate, cellulose derivatives such as microcrystalline cellulose, silicified microcrystalline cellulose; starch derivatives such as pregelatinized starch and corn starch; phosphates such as dibasic calcium phosphate; mannitol, maltitol, dextrose, calcium phosphates, or combinations thereof.

In a non-limiting embodiment, pharmaceutically acceptable stabilizing agents are selected from the group consisting of anhydrous lactose, microcrystalline cellulose, starches, mannitol, maltitol, dextrose, calcium phosphates, or combinations thereof.

Pharmaceutically acceptable disintegrants include, but are not limited to, cellulose derivatives, for example, croscarmellose sodium, hydroxypropyl celluloses, carboxymethyl celluloses, microcrystalline cellulose; povidone derivatives such as crospovidone, plasdones; starch derivatives such as pregelatinized starch, sodium starch glycolate, and corn starch; or combinations thereof.

Examples of pharmaceutically acceptable glidants include cellulose derivatives such as carboxymethyl celluloses, microcrystalline cellulose; starch derivatives, for example, pregelatinized starch and corn starch; silicate derivatives, for example, magnesium silicate, magnesium trisilicate, and talc.

Examples of pharmaceutically acceptable lubricants include, but are not limited to, stearate derivatives such as magnesium stearate, zinc stearate, calcium stearate, stearic acid, monostearates, and stearyl fumarate; sulfated derivatives such as magnesium lauryl sulfate.

As to the first phase comprising rosuvastatin, as indicated above, it is in the form of a coated tablet.

Said rosuvastatin tablet further comprises at least one of a diluent, stabilizing agent, disintegrant, glidant, and lubricant.

In a non-limiting embodiment, said diluent is selected from the group consisting of lactose monohydrate; cellulose derivatives including carboxymethyl cellulose, mannitol, maltitol, dextrose, microcrystalline cellulose, anhydrous calcium phosphate, corn starches, and combinations thereof. The stabilizing agent is selected from the group consisting of phosphate derivatives including anhydrous dibasic calcium phosphate, aluminum magnesium silicate, anhydrous lactose, microcrystalline cellulose, starches, mannitol, maltitol, dextrose, and combinations thereof. Said disintegrant is selected from the group consisting of croscarmellose sodium, cellulose derivatives including hydroxypropyl celluloses, carboxymethyl celluloses, microcrystalline cellulose; povidone derivatives including plasdones; starch derivatives including pregelatinized starch, sodium glycolate starch, and corn starch. Said glidant is selected from the group consisting of colloidal silicon dioxide; cellulose derivatives including carboxymethyl celluloses, microcrystalline cellulose; starch derivatives including pregelatinized starch and corn starch; silicate derivatives including magnesium silicate, magnesium trisilicate; talc; and combinations thereof. The lubricant is selected from the group consisting of stearate derivatives including zinc stearate, magnesium stearate, calcium stearate, stearic acid, monostearates, stearyl fumarate; sulfated derivatives including magnesium lauryl sulfate and sodium lauryl sulfate; talc; and combinations thereof.

As to the coating, it acts as a protective barrier between the active ingredients and as a barrier against moisture, retaining the stability of a composition. In a non-limiting embodiment, the coating is selected from the group consisting of cellulose derivatives such as hydroxypropyl methylcellulose, hydroxypropyl cellulose, carboxymethyl celluloses; polyvinyl derivatives such as polyvinyl acetate; polyethylene glycol, povidones in all K grades and derivatives thereof. In one embodiment, the coating is polyvinyl acetate; in a preferred embodiment, the coating is Opadry, such as Opadry AMB II white 88A180021. To incorporate the coating, as will be described below, water is used as a solvent; however, it evaporates during the process.

In a more preferred embodiment, the first phase comprising rosuvastatin further comprises two lubricants, wherein the first lubricant is talc, and the second lubricant is zinc stearate.

In an even more preferred embodiment, the first phase comprising rosuvastatin further comprises:
- rosuvastatin or a pharmaceutically acceptable salt thereof as an active ingredient in a dose of from 10 mg to 20 mg rosuvastatin;
- lactose monohydrate as a diluent in an amount of from 40% to 80% by weight of the tablet;
- dibasic calcium phosphate as a stabilizing agent in an amount of from 0.26% to 2% by weight of the tablet;
- sodium croscarmellose as a disintegrant in an amount of from 0.5% to 10% by weight of the tablet;
- colloidal silicon dioxide as a glidant in an amount of from 0.08% to 2% by weight of the tablet;
- talc as a first lubricant in an amount of from 0.5% to 10% by weight of the tablet;
- zinc stearate as a second lubricant in an amount of from 0.5% to 10% by weight of the tablet; and
- a polyvinyl acetate coating in an amount of from 0.5% to 10% by weight of the tablet.

In a preferred embodiment, the rosuvastatin tablet comprises a dose of 10 mg rosuvastatin or a pharmaceutically acceptable salt thereof.

In another preferred embodiment, the rosuvastatin tablet comprises a dose of 20 mg rosuvastatin or a pharmaceutically acceptable salt thereof.

As to the pharmaceutically acceptable salt of rosuvastatin, in a preferred embodiment, rosuvastatin calcium is employed. In this regard, a person skilled in the art will understand that adjustments must be made to the amount of rosuvastatin calcium employed to obtain the desired dose.

Based on the above, in a preferred embodiment, the rosuvastatin in the tablet is preferably in its calcium form, wherein 10.40 mg rosuvastatin calcium is equivalent to a dose of 10 mg rosuvastatin.

In another preferred embodiment, the rosuvastatin in the tablet is preferably in its calcium form, wherein 20.80 mg rosuvastatin calcium is equivalent to a dose of 20 mg rosuvastatin.

In a preferred embodiment, the amount of the active ingredient rosuvastatin is adjusted according to titration.

In a preferred embodiment, the tablet coating is in an amount of from 0.5% to 10% by weight of the tablet.

On the other hand, in a preferred embodiment, the second phase comprising acetylsalicylic acid, which is in the form of granules, further comprises at least one of a diluent, a binder, a wetting agent, a glidant, and a lubricant.

In a non-limiting embodiment, the binder is selected from the group consisting of glyceryl dibehenate; povidones and derivatives thereof; cellulose derivatives including carbomers, hydroxypropyl cellulose, carboxymethyl cellulose; starch derivatives including pregelatinized starch and corn starch; polyethylene glycol in its different grades; poloxamer; and combinations thereof. The wetting agent is selected from the group consisting of sodium lauryl sulfate; poloxamers; polyoxyethylene castor oil derivative; benzalkonium chloride; benzetonium chloride; polyoxyethylene alkyl ethers; sorbitan polyoxyethylene fatty acid esters; and combinations thereof. The glidant is selected from the group consisting of colloidal silicon dioxide; cellulose derivatives including carboxymethyl cellulose and microcrystalline cellulose; starch derivatives including pregelatinized starch and corn starch; silicate derivatives including magnesium silicate and magnesium trisilicate; talc; and combinations thereof. The lubricant is selected from the group consisting of stearate derivatives including magnesium stearate, zinc stearate, calcium stearate, stearic acid, monostearates, and stearyl fumarate; sulfated derivatives including magnesium lauryl sulfate and sodium lauryl sulfate; talc; and combinations thereof. The diluent is selected from the group consisting of starch derivatives including pregelatinized starch and corn starch; cellulose derivatives including microcrystalline cellulose, carbomers, hydroxypropyl cellulose, and carboxymethyl cellulose; polyethylene glycol in its different grades; poloxamer; and combinations thereof.

In a more preferred embodiment, the acetylsalicylic acid granules comprise two diluents, wherein the first diluent is pregelatinized starch, and the second diluent is microcrystalline cellulose.

In an even more preferred embodiment, the acetylsalicylic acid granules comprise:
- acetylsalicylic acid as an active ingredient in a dose of 100 mg;
- pregelatinized starch as a first diluent in an amount of from 5% to 75% by weight of the granules;
- glyceryl dibehenate as a binder in an amount of from 0.4% to 4% by weight of the granules;
- microcrystalline cellulose as a second diluent in an amount of from 5% to 90% by weight of the granules;
- sodium lauryl sulfate as a wetting agent in an amount of from 0.02% to 4% by weight of the granules;
- colloidal silicon dioxide as a glidant in an amount of from 0.08% to 2% by weight of the granules; and
- zinc stearate as a lubricant in an amount of from 0.1% to 1%.

In a preferred embodiment, the acetylsalicylic acid granules comprise acetylsalicylic acid preferably in its free form. In this regard, a person skilled in the art will understand that adjustments must be made to the amount of acetylsalicylic acid to obtain the desired equivalent dose; for example, amounts of 100 ± 0.5 mg can be used to obtain a dose of 100 mg.

In one embodiment, the pharmaceutical composition of the present invention is a solid form, preferably a capsule.

The pharmaceutical composition as described above is notable for its physical, chemical, and biological stability, as well as for its controlled drug release and low cost.

Surprisingly, the pharmaceutical composition of the present application was found to be highly stable, since, in a 6-month accelerated stability study, the pharmaceutical composition of the present invention was found to retain its chemical and physical characteristics within the established specifications, maintaining its quality, efficacy, and safety.

Additionally, the bioavailability of the active ingredients rosuvastatin and acetylsalicylic acid contained in the pharmaceutical composition of the present invention was found to be comparable to the concomitant administration of reference medicaments in human subjects, thus demonstrating the effectiveness of the composition. Furthermore, the composition of the present invention is safe as no serious adverse events occurred; in fact, the number of adverse events observed in a clinical study was higher for the concomitant administration of monodrugs.

The pharmaceutical composition of the present invention is useful in the secondary prevention of cardiovascular events. In this sense, secondary prevention refers to reduced risks of death or disability caused by recurrent coronary and cerebrovascular disease.

Likewise, there is evidence that the combination of acetylsalicylic acid and rosuvastatin is safe; in addition, it is an effective replacement therapy for mono-components in the secondary prevention of secondary events. The combined use of said drugs is considered part of the comprehensive management of patients with cardiovascular disease in secondary prevention and is supported by various international guidelines, such as those of the American Heart Association and American College of Cardiology Foundation (AHA/ACCF) and the European Society of Cardiology (ESC). The magnitude of benefit of the combination is associated with the anti-inflammatory properties of the drugs, which reduce cholesterol plaque, inflammation, platelet aggregation activation, and ultimately, cardiovascular events.

Accordingly, the present invention further provides a method for the secondary prevention of cardiovascular events as replacement therapy, which comprises administering a pharmaceutical composition according to the present invention, wherein the treatment is preferably intended for adult patients adequately controlled with the mono-components administered concurrently at equivalent therapeutic doses. Advantageously, the composition of the present invention provides better patient adherence to treatment, as it reduces the number of doses to be consumed while maintaining the effectiveness of the co-administered drugs.

Furthermore, considering that rosuvastatin is a lipid-lowering agent, the present invention also provides a method for treating hyperlipidemia and cardiovascular diseases. In a non-limiting embodiment, hyperlipidemia and cardiovascular disease are selected from the group consisting of hyperlipidemia, hyperlipoproteinemia, hypercholesterolemia, hypertension, angina pectoris, heart attacks, and aneurysms, comprising the administration of a pharmaceutical composition according to the present invention. In one embodiment, the pharmaceutical composition of the present invention participates in the regulation of plasma low-density lipoprotein (LDL), high-density lipoprotein (HDL), triglyceride, cholesterol concentration.

In a preferred embodiment, the treatment method comprises administering a composition according to the present invention once a day.

The present invention also discloses an optimized process that allows a pharmaceutical composition as described above to be manufactured.

The process for manufacturing the pharmaceutical composition as described above comprises the following stages:
a) providing a first phase comprising rosuvastatin, wherein the first phase comprising rosuvastatin is in the form of a coated tablet;
b) providing a second phase comprising acetylsalicylic acid, wherein the second phase comprising acetylsalicylic acid is in the form of granules; and
c) encapsulating the first phase comprising rosuvastatin and the second phase comprising acetylsalicylic acid.

The manufacture of the first phase comprising rosuvastatin involves the following steps:
- mixing rosuvastatin with a glidant, a disintegrant, a stabilizing agent, a diluent, and a first lubricant;
- lubricating the above mixture with at least one lubricant;
- compressing the lubricated mixture to obtain a rosuvastatin tablet;
- coating the tablet.

In a non-limiting embodiment, the diluent is lactose monohydrate, the stabilizing agent is dibasic calcium phosphate, the disintegrant is croscarmellose sodium, the glidant is colloidal silicon dioxide, the solvent is purified water, the first lubricant is talc, the second lubricant is zinc stearate, and the coating is polyvinyl acetate.

Additionally, for incorporating the coating, it is dispersed in water, which evaporates during the process.

The second phase comprising acetylsalicylic acid, which corresponds to acetylsalicylic acid granules, wherein said granules have no enteric coating.

To manufacture the acetylsalicylic acid granules, the following steps are carried out:
- Mixing the acetylsalicylic acid with a wetting agent, a first diluent, and a binder.
- granulating and heating the above mixture;
- cooling the mixture and adding a glidant and a second diluent;
- lubricating the mixture with a lubricant.

In a non-limiting embodiment, the binder is glyceryl dibehenate, the wetting agent is lauryl sulfate, the glidant is colloidal silicon dioxide, the lubricant is zinc stearate, the first diluent is pregelatinized starch, and the second diluent is microcrystalline cellulose.

In one preferred embodiment, the manufacture of acetylsalicylic acid granules involves a melt granulation process wherein a solid binder is used at room temperature but melts at relatively low temperatures. Said binder can be added to the preheated powder mixture either in the molten form or in the solid form along with the remaining mixture components, so that granulation takes place in a single step by melting the binder within the mixture. After melting, the binder acts as a liquid binder for the powder particles in a similar way to the liquid added in wet processes.

The melt granulation process avoids dust formation, eliminates the drying step, thereby shortening the granulation process, and avoids the use of solvents and the potential problems that arise therefrom (hydrolysis of the active ingredient).

In this particular case, melt granulation allows a protective barrier to be created between drugs, thus ensuring their stability.

Since glyceryl dibehenate has a melting point between 65 °C and 85 °C, the process requires reaching a temperature of 85 °C, at which the binder melts. Temperature control is critical, as it is a crucial factor in the drug dissolution and granulation characteristics that ensure adequate flow for the next encapsulating stage.

Among the characteristics of acetylsalicylic acid are high solubility, high permeability, and sensitivity to light, which characteristics can be controlled through the granulation process with glyceryl dibehenate (binder) which, by being hydrophobic, creates an isolating barrier that prevents interaction with rosuvastatin. Likewise, the inclusion of the wetting agent sodium lauryl sulfate in the granulation process allows pores to be created in the binder barrier, ensuring proper dissolution of acetylsalicylic acid.

On the other hand, the encapsulation stage comprises putting the first phase comprising rosuvastatin and the second phase comprising acetylsalicylic acid into a gelatin capsule.

Due to the novel process and formulation of the medicament, it was possible to obtain a product with demonstrably improved stability through studies conducted according to current regulatory guidelines, in which quality attributes were met during the assessment period. Stability testing is a means of comparing different formulations, packaging materials, or manufacturing processes in short-term experiments. Once the final formulation and manufacturing process have been established, the manufacturer conducts a series of stability tests to predict the product or drug stability in this case and determine shelf-life and storage conditions thereof.

The initial results and results under accelerated conditions for determining the stability of compositions of the present invention for the manufacture of a medicament useful in the treatment and prevention of cardiovascular diseases under conditions of 40°C/75% RH, are shown below in Example 2.

Technological development consisted of a number of assessments defining the formulation and process as described below. The skilled person in the art will realize that multiple variations and embodiments are possible in performing the present invention without departing from the spirit and scope thereof to ensure proper product function and to meet the required quality characteristics.

The present invention will be better understood from the following examples, which are presented solely for illustrative purposes to allow a thorough understanding of the preferred embodiments of the present invention, without implying that there are no other embodiments not illustrated that can be put into practice based on the detailed description.

### EXAMPLES

### Example 1. Process for manufacturing a pharmaceutical composition according to one embodiment of the invention

Two pharmaceutical compositions comprising rosuvastatin and acetylsalicylic acid were manufactured, the first one having a dose of 10 mg rosuvastatin/100 mg acetylsalicylic acid, the qualitative and quantitative formula of which is detailed in Table 1, and the second one having a dose of 20 mg rosuvastatin/100 mg acetylsalicylic acid, the qualitative and quantitative formula of which is detailed in Table 2.

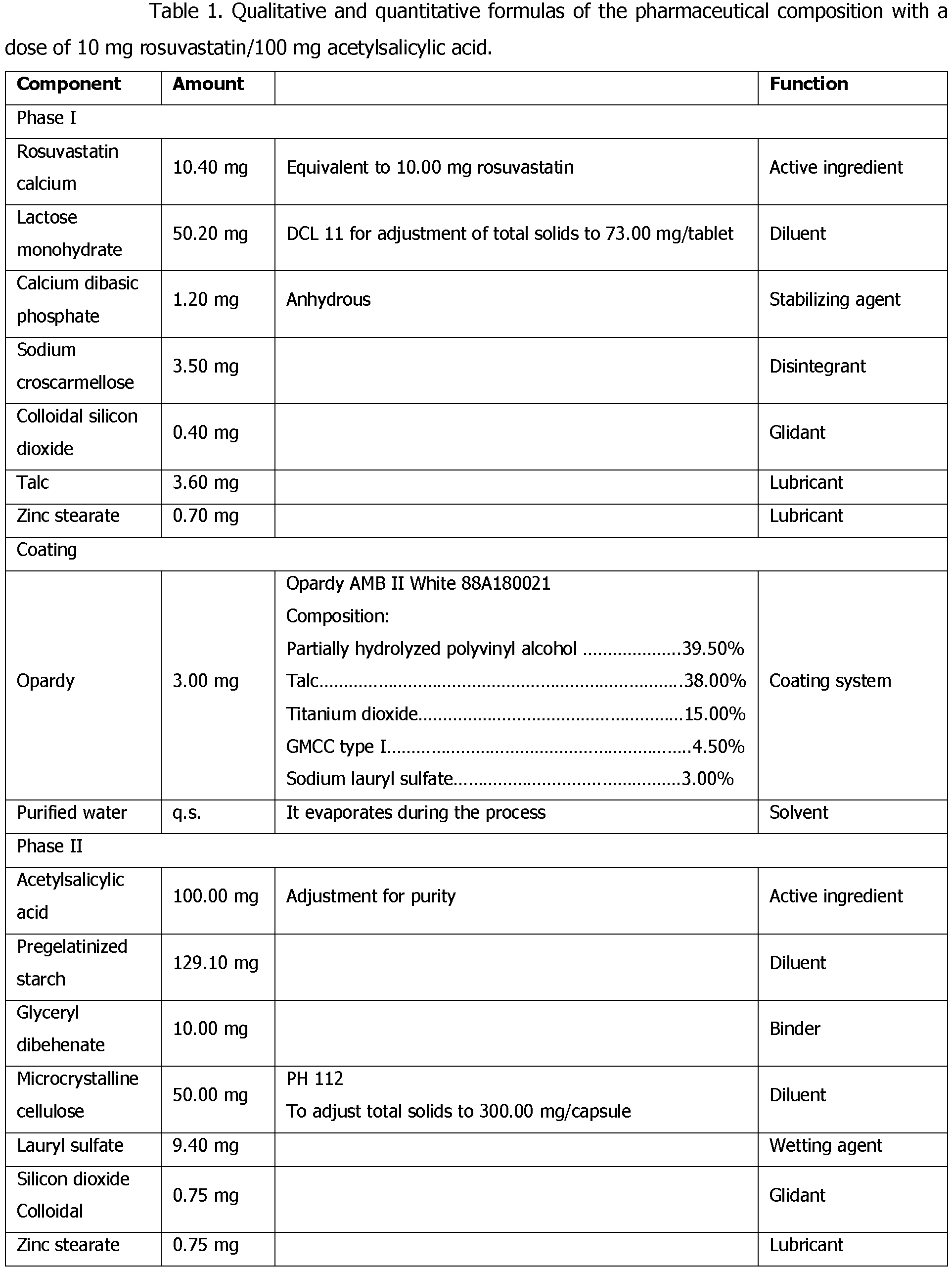

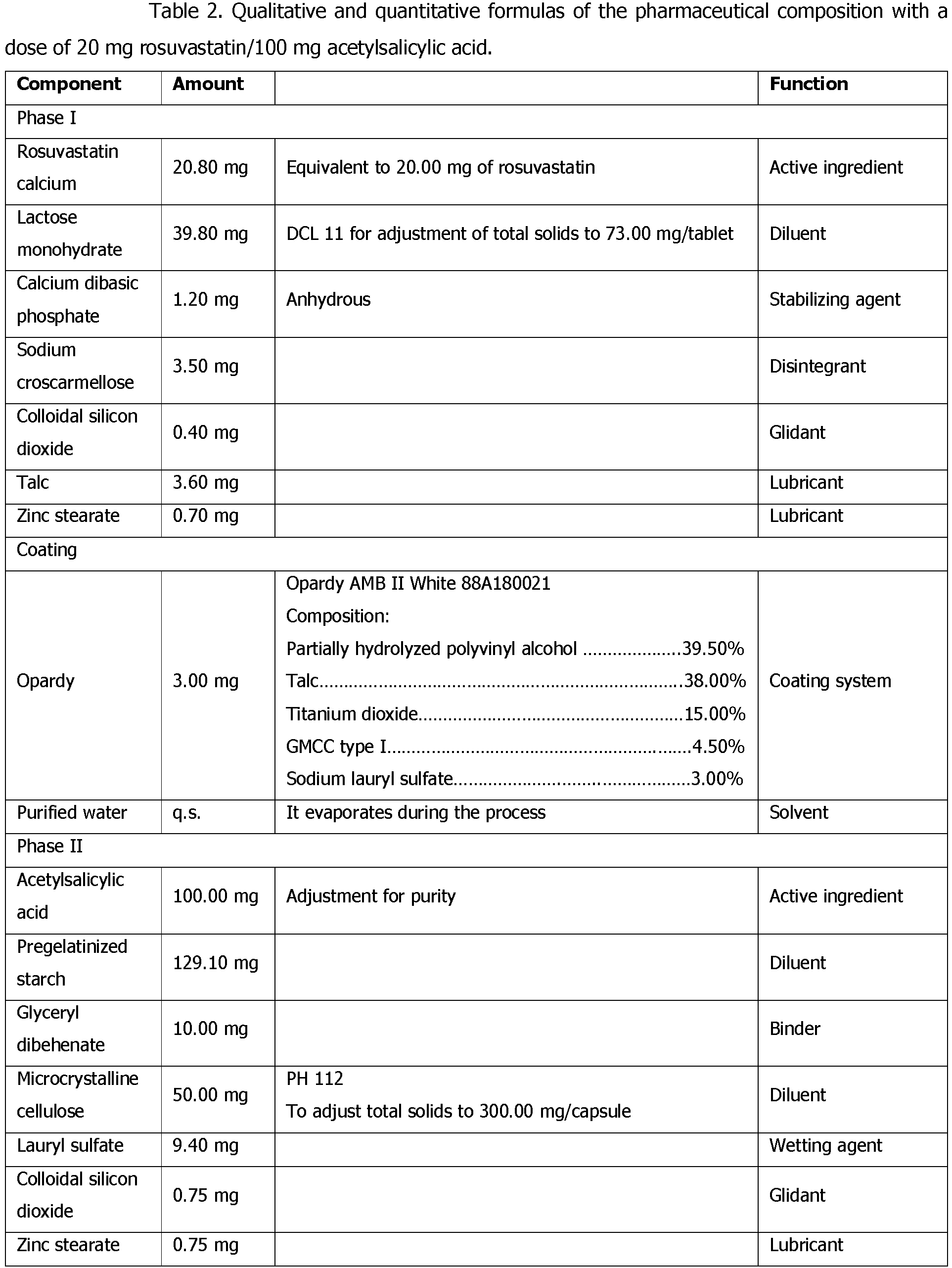

To manufacture the pharmaceutical compositions according to one embodiment of the present invention as described above, a process generally consisting of three stages described below was carried out:

### I. Preparation of a first phase comprising rosuvastatin:

The aim of this stage is to prepare a first phase comprising rosuvastatin, for which the following steps were carried out:
1. Rosuvastatin calcium (active ingredient) was mixed with a glidant (colloidal silicon dioxide), a disintegrant (croscarmellose sodium), and a stabilizing agent (anhydrous dibasic calcium phosphate) for approximately 8 minutes. Excipients were previously sieved.
2. A sieving operation was performed on the mixture using a 0.045-inch (1.143 mm) mesh.
3. The sifted material from step No. 2 was mixed with a diluent (lactose monohydrate DCL 11) for approximately 5 minutes.
4. The mixture from step No. 3 was mixed with a first lubricant (talc) previously sieved for approximately 3 minutes.
5. The mixture from step No. 4 was mixed with a second lubricant (zinc stearate) for approximately 3 minutes.
6. The lubricated mixture obtained in step No. 5 was compressed into 70 ± 7.0 mg cores, with a hardness of 3.0-8.0 kp and a disintegration time of less than 5 minutes.
7. The cores obtained in step No. 6 were coated with a polyvinyl acetate coating, which is Opardy AMB II white 88A180021, which was previously dispersed in water.

The result of the above steps is a white colored, biconvex, coated tablet plain on both sides with an average weight: 73.0 mg ± 7.0 mg; a thickness: 3.0 mm to 3.6 mm; and a disintegration time of no more than 5 min in water at 37 °C ± 2 °C.

### II. Preparation of a second phase comprising acetylsalicylic acid:

The aim of this stage is to prepare a second phase comprising acetylsalicylic acid, for which the following steps were carried out:
1. Acetylsalicylic acid (active ingredient), a wetting agent (sodium lauryl sulfate), a portion (approximately 38.7%) of the first diluent (pregelatinized starch), and a binder (glyceryl dibehenate) were mixed for approximately 3 minutes in a granulation equipment.
2. The granulation equipment was heated for approximately 15 min at a temperature of approximately 85 °C.
3. A granulation process was carried out until a temperature of approximately 65°C to 70°C was reached.
4. The remainder (approximately 61.3%) of the first diluent (pregelatinized starch) was added.
5. The granulation equipment was cooled until a temperature of approximately 30 °C to 35 °C was reached.
6. The particle size was reduced by sieving the granules from step No. 5 through a 0.050-inch mesh.
7. A glidant (colloidal silicon dioxide) and a second diluent (microcrystalline cellulose PH112) were added and mixed for approximately 5 minutes.
8. A lubricant (zinc stearate) was added and mixed for approximately 3 minutes.

### III. Encapsulation

To encapsulate, and therefore, prepare a pharmaceutical composition according to the present invention, a size #0 capsule with opaque white body and opaque red cap was used. To this end, said phase I comprising rosuvastatin and said phase II comprising acetylsalicylic acid were put into said gelatin capsule.

Average weight of capsule (capsule + powder) was 396.0 mg ± 30.0 mg; average weight of contents (granules + tablet) was 373.0 mg ± 30.0 mg; average weight of filled capsule was 469.0 mg ± 30.0 mg; disintegration time was less than 15 min in water at 37 °C ± 2.0 °C.

Finally, the pharmaceutical composition was packaged in a primary container consisting of a cold-formed aluminum foil blister pack 176 µm - aluminum foil 25 µm, which provides airtightness; the secondary container is a white cardboard box.

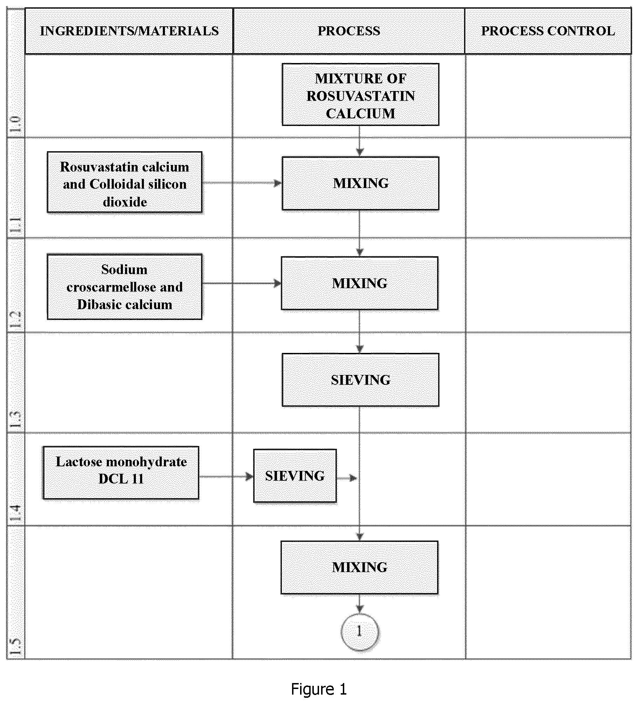

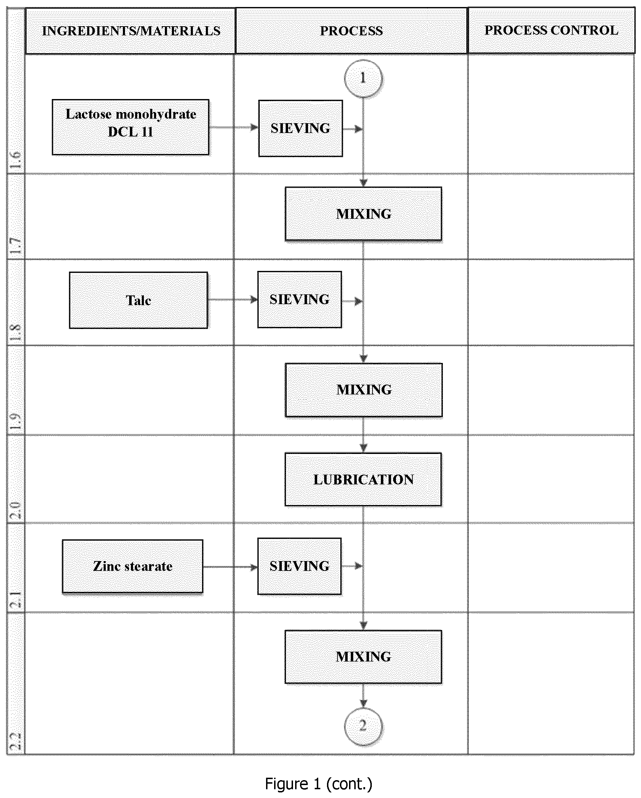

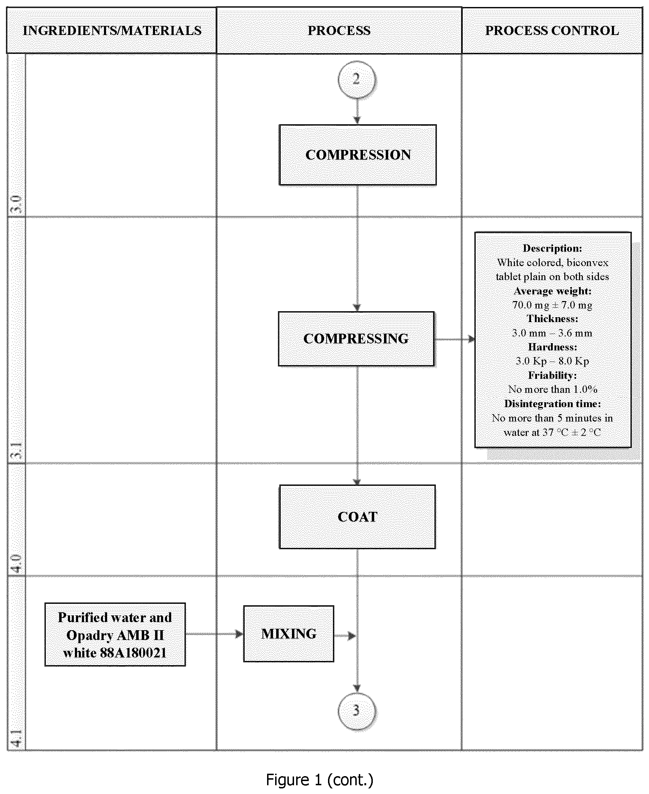

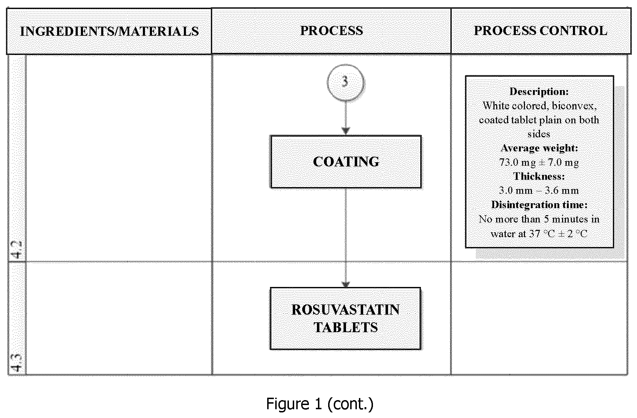

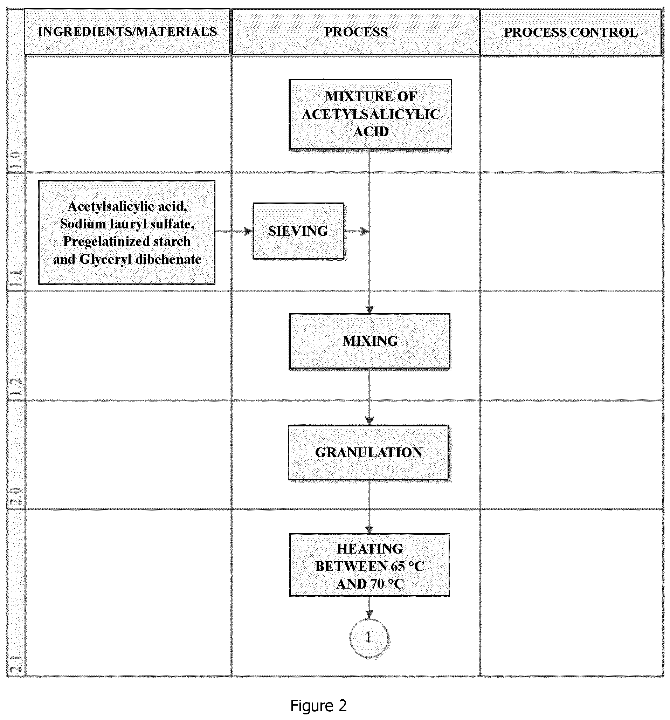

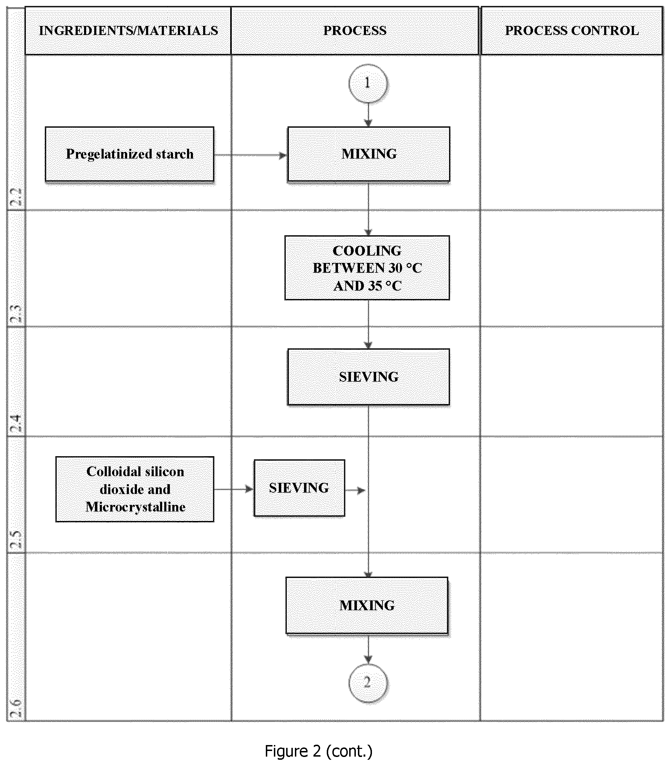

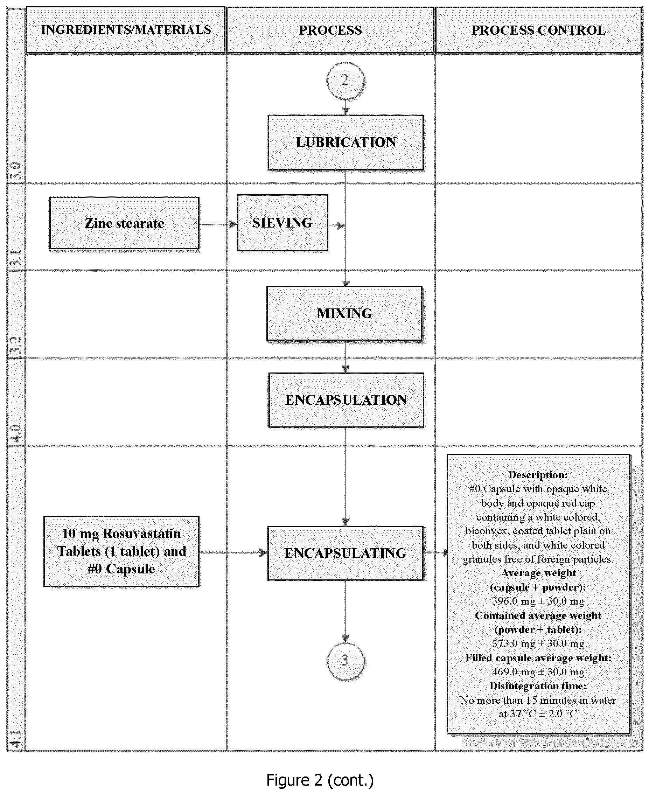

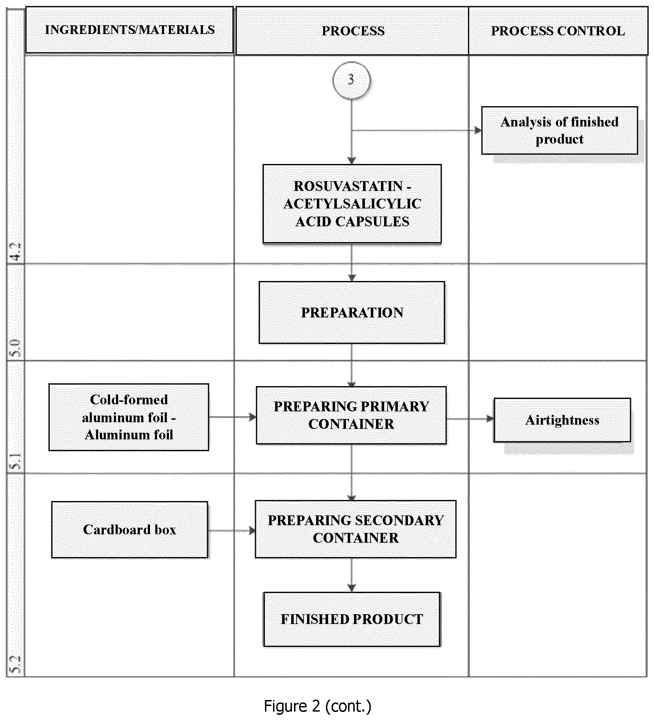

The detailed process is described in Figures 1 and 2. Specifically, Figure 1 is a flowchart of the Stage I for preparing the first phase comprising rosuvastatin, while Figure 2 is a flowchart of Stages II and III, i.e., for preparation of the second phase comprising acetylsalicylic acid, and for encapsulation.

### Example 2. Stability of a pharmaceutical composition according to a preferred embodiment of the present invention.

The aim of this study was to assess the stability of a pharmaceutical composition according to a preferred embodiment of the present invention, particularly a composition as described in Example 1, Table 1 (dose of 10 mg rosuvastatin/100 mg acetylsalicylic acid) under accelerated stability conditions (40 °C ± 2 °C / 75% ± 2% relative humidity) for 6 months.

The study began on July 30, 2021, and ended on January 30, 2022, in compliance with Section 11 of Mexican Official Standard NOM-059-SSA1-2015, Good Manufacturing Practices for Medicines, and in accordance with Section 7.1 of NOM-073-SSA1-2015, Stability of Drugs and Medicines, as well as Herbal Remedies.

During the study, an assessment was performed on three batches (DFF2106-17, DFF2106-18, and DFF2106-19) of the pharmaceutical composition described in Table 1 (dose of 10 mg rosuvastatin/100 mg acetylsalicylic acid) and packaged in a primary container consisting of a cold-formed aluminum foil blister pack 176 µm - aluminum foil 25 µm; the secondary container being a white cardboard box.

Initial sampling was performed at the end of the first month (August 30, 2021), third month (October 30, 2021), and six months (January 30, 2022). The following samples were considered for each analysis period:
- 40 blister packs for physicochemical testing.
- 32 blister packs for microbiological testing for the initial and final periods.

The parameters analyzed were product description, rosuvastatin content, acetylsalicylic acid content, rosuvastatin dissolution, acetylsalicylic acid dissolution, organic impurities in rosuvastatin, salicylic acid limit, microbial limits (initial and final periods), and water content (tablet and granules).

Tables 3, 4, and 5 show the results obtained for the three batches assessed.

**Table 3. Results of stability study for batch DFF2106-17**

| **Determination** | **Specification** | **Sample No.** | **Initial July 30, 2021** | **1 month Aug 30, 2021** | **3 months Oct 30, 2021** | **6 months Jan 30, 2022** |
|---|---|---|---|---|---|---|
| | Rosuvastatin 90.0% to 110.0% | M1 | 97.25% | 98.22% | 95.99% | 96.42% |
| | | M2 | 98.01% | 94.61% | 95.67% | 96.47% |
| | | M3 | 97.23 | 97.70 | 95.57 | 96.43% |
| Rosuvastatin Content | | M_{avg} | 97.5 | 97.8% | 95.7 | 96.4% |
| | | CV | 0.46 | 0.34 | 0.23 | 0.02 |
| | Rosuvastatin 10.0 mg/capsule | M1 | 9.73 | 9.82 | 9.60 | 9.64 |
| | | M2 | 9.80 | 9.76 | 9.57 | 9.65 |
| | | M3 | 9.72 | 9.77 | 9.56 | 9.64 |
| | | M_{avg} | 9.8 | 9.8 | 9.6 | 9.6 |
| | Acetylsalicylic acid 90.0% to 110.0% | M1 | 107.03 | 105.89 | 102.27 | 97.30 |
| | | M2 | 106.48 | 106.03% | 102.08% | 97.32% |
| | | M3 | 106.58 | 105.94 | 101.93% | 97.17% |
| Acetylsalicylic Acid content | | M_{avg} | 106.7 | 106.0% | 102.1% | 97.3% |
| | | CV | 0.27 | 0.07 | 0.16 | 0.08 |
| | Acetylsalicylic acid 100.0 mg/capsule | M1 | 107.03 | 105.89 | 102.27 | 97.30 |
| | | M2 | 106.48 | 106.03 | 102.08 | 97.32 |
| | | M3 | 106.58 | 105.94 | 101.93 | 97.17 |
| | | M_{avg} | 106.7 | 106.0 | 102.1 | 97.3 |
| Rosuvastatin Dissolution | Rosuvastatin Q = 75.0% in 30 inutes | M1 | 97.72 | 91.60 | 95.59 | 93.25 |
| | | M2 | 102.24% | 94.00% | 89.72% | 90.61% |
| | | M3 | 99.52% | 101.35% | 98.60% | 83.32% |
| | | M4 | 96.52 | 96.40 | 95.80 | 96.39% |
| | | M5 | 97.68% | 101.53% | 98.39 | 93.43% |
| | | M6 | 96.76 | 96.21% | 103.75% | 99.46% |
| | | M_{avg} | 98.4 | 96.9% | 97.8 | 92.7% |
| | | CV | 2.19 | 4.09 | 4.46 | 5.96 |
| Acetylsalicylic Acid Dissolution | Acetylsalicylic acid Q = 70.0% in 45 minutes | M1 | 103.43 | 93.79 | 89.52 | 79.72 |
| | | M2 | 93.74% | 91.51 | 92.65% | 81.79% |
| | | M3 | 102.27% | 86.88% | 91.73% | 76.08% |
| | | M4 | 99.08% | 89.42 | 85.11% | 85.36% |
| | | M5 | 101.78% | 85.34% | 93.07 | 77.18 |
| | | M6 | 95.53% | 88.32 | 94.35 | 83.16% |
| | | M_{avg} | 99.3% | 89.2 | 91.1 | 80.6% |
| | | CV | 3.96 | 3.46 | 3.66 | 4.42 |
| Organic impurities in Rosuvastatin | Rosuvastatin ketone: No more than 2.10% | M1 | 0.15% | 0.25% | 0.27 | 0.44 |
| | Rosuvastatin lactone: No more than 3.80% | M1 | 0.09 | 0.27 | 0.54 | 3.47 |
| | Rosuvastatin ethyl ester: No more than 0.50% | M1 | Not detected | Not detected | Not detected | Not detected |
| | Any non-specific degradation product: No more than 0.20% | M1 | 0.03 | 0.04 | 0.01 | 0.03 |
| | Total degradation products: No more than 3.60% | M1 | 0.09 | 0.08 | 0.06 | 0.18 |
| Salicylic acid imit | No more than .00% | X20 | 0.28 | 0.39 | 0.38 | 1.56% |
| Microbial limits | Aerobic mesophilic count: No more than 10³ CFU/g | X20 | Less than 10 CFU/g | Not applicable | Less than 10 CFU/g | Less than 10 CFU/g |
| | Filamentous fungi and yeasts: No more than 10² CFU/g | | Less than 10 CFU/g | Not applicable | Less than 10 CFU/g | Less than 10 CFU/g |
| | Absence of pathogens: Absence of *Escherichia coli* | | Absence of *Escherichia coli* | Not applicable | Absence of *Escherichia coli* | Absence of *Escherichia coli* |
| Water content | Tablet: Not more than 15.00% | X20 | 4.55 | 4.67 | 4.77 | 3.98 |
| | Granules: No more than 15.00% | | 4.16 | 4.33 | 4.11 | 2.93 |

**Table 4. Results of stability study for batch DFF2106-18**

| **Determination** | **Specification** | **Sample No.** | **Initial July 30, 2021** | **1 month Aug 30, 2021** | **3 months Oct 30, 2021** | **6 months Jan 30, 2022** |
|---|---|---|---|---|---|---|
| | Rosuvastatin 90.0% to 110.0% | M1 | 98.06 | 95.40% | 97.45% | 97.53% |
| | | M2 | 98.59 | 96.57% | 97.36% | 97.48% |
| | | M3 | 98.09% | 96.17% | 97.25% | 97.58% |
| Rosuvastatin Content | | M_{avg} | 98.3 | 96.1% | 97.4% | 97.5% |
| | | CV | 0.30 | 0.62 | 0.10 | 0.05 |
| | Rosuvastatin 10.0 mg/capsule | M1 | 9.81 | 9.54 | 9.75 | 9.75 |
| | | M2 | 9.86 | 9.66 | 9.74 | 9.75 |
| | | M3 | 9.81 | 9.62 | 9.73 | 9.76 |
| | | M_{avg} | 9.8 | 9.6 | 9.7 | 9.8 |
| Acetylsalicylic acid content | Acetylsalicylic acid 90.0% to 110.0% | M1 | 105.00 | 101.93 | 102.55 | 97.10 |
| | | M2 | 106.80% | 102.45% | 103.02% | 97.11% |
| | | M3 | 103.55 | 102.36% | 102.53% | 97.12% |
| | | M_{avg} | 105.1 | 102.3% | 102.7% | 97.1% |
| | | CV | 1.55 | 0.27 | 0.27 | 0.01 |
| | Acetylsalicylic acid 100.0 mg/capsule | M1 | 105.00 | 101.93 | 102.55 | 97.10 |
| | | M2 | 106.80 | 102.45 | 103.02 | 97.11 |
| | | M3 | 103.55 | 102.36 | 102.53 | 97.12 |
| | | M_{avg} | 105.1 | 102.3 | 102.7 | 97.1 |
| Rosuvastatin Dissolution | Rosuvastatin Q = 75.0% in 30 minutes | M1 | 95.25 | 94.86 | 92.74 | 90.55 |
| | | M2 | 98.07% | 95.89% | 93.02% | 94.51% |
| | | M3 | 96.54 | 100.67% | 96.14 | 92.49% |
| | | M4 | 100.61% | 98.93 | 96.70 | 100.52% |
| | | M5 | 100.70 | 100.83% | 101.18% | 95.08% |
| | | M6 | 97.59% | 100.89 | 98.83% | 93.70% |
| | | M_{avg} | 98.1 | 98.7 | 96.4 | 94.5% |
| | | CV | 2.23 | 2.71 | 3.40 | 3.57 |
| Acetylsalicylic Acid Dissolution | Acetylsalicylic acid Q = 70.0% in 45 minutes | M1 | 82.23 | 90.94 | 93.24 | 82.94 |
| | | M2 | 82.78% | 94.56 | 90.27% | 79.71% |
| | | M3 | 96.22% | 89.99% | 87.68 | 85.33% |
| | | M4 | 89.64% | 94.39 | 79.32 | 88.94% |
| | | M5 | 83.82 | 89.16% | 90.81 | 86.94% |
| | | M6 | 79.19 | 91.63 | 87.38 | 82.04% |
| | | M_{avg} | 85.7 | 91.8 | 88.1% | 84.3% |
| | | CV | 7.25 | 2.45 | 5.47 | 4.02 |
| Organic impurities in Rosuvastatin | Rosuvastatin ketone: No more than 2.10% | M1 | 0.16 | 0.23 | 0.31 | 0.44 |
| | Rosuvastatin lactone: No more than 3.80% | M1 | 0.09 | 0.27 | 0.51 | 3.10 |
| | Rosuvastatin ethyl ester: No more than 0.50% | M1 | Not detected | Not detected | Not detected | Not detected |
| | Any non-specific degradation product: No more than 0.20% | M1 | 0.04 | 0.03 | 0.01 | 0.03 |
| | Total degradation products: No more than 3.60% | M1 | 0.10 | 0.07 | 0.06 | 0.16 |
| Salicylic acid limit | No more than 2.00% | X20 | 0.26 | 0.37 | 0.36 | 1.51 |
| Microbial limits | Aerobic mesophilic count: No more than 10³ CFU/g | X20 | Less than 10 CFU/g | Not applicable | Less than 10 CFU/g | Less than 10 CFU/g |
| | Filamentous fungi and yeasts: No more than 10² CFU/g | | Less than 10 CFU/g | Not applicable | Less than 10 CFU/g | Less than 10 CFU/g |
| | Absence of pathogens: Absence of *Escherichia coli* | | Absence of *Escherichia coli* | Not applicable | Absence of *Escherichia coli* | Absence of *Escherichia coli* |
| Water content | Tablet: No more than 15.00% | X20 | 4.54 | 4.76 | 4.87 | 4.31 |
| | Granules: No more than 15.00% | | 3.95 | 4.21 | 4.24 | 3.08 |

**Table 5. Results of stability study for batch DFF2106-19**

| **Determination** | **Specification** | **Sample No.** | **Initial July 30, 2021** | **1 month Aug 30, 2021** | **3 months October 30, 2021** | **6 months Jan 30, 2022** |
|---|---|---|---|---|---|---|
| Rosuvastatin Content | Rosuvastatin 90.0% to 110.0% | M1 | 98.20% | 98.37% | 96.72% | 96.98% |
| | | M2 | 98.48% | 98.43% | 96.72 | 97.13% |
| | | M3 | 98.30 | 97.44% | 96.86 | 97.11% |
| | | M_{avg} | 98.3 | 98.1% | 96.8 | 97.1% |
| | | CV | 0.14 | 0.57 | 0.09 | 0.08 |
| | Rosuvastatin 10.0 mg/capsule | M1 | 9.82 | 9.84 | 9.67 | 9.70 |
| | | M2 | 9.85 | 9.84 | 9.67 | 9.71 |
| | | M3 | 9.83 | 9.74 | 9.69 | 9.71 |
| | | M_{avg} | 9.8 | 9.8 | 9.7 | 9.7 |
| | Acetylsalicylic acid 90.0% to 110.0% | M1 | 105.34 | 103.94% | 104.76 | 96.63 |
| | | M2 | 105.25% | 104.39% | 104.89% | 96.40% |
| | | M3 | 104.96 | 104.53% | 105.10 | 96.56% |
| Acetylsalicylic acid content | | M_{avg} | 105.2 | 104.3% | 104.9% | 96.5% |
| | | CV | 0.19 | 0.29 | 0.16 | 0.12 |
| | Acetylsalicylic acid 100.0 mg/capsule | M1 | 105.34 | 103.94 | 104.76 | 96.63 |
| | | M2 | 105.25 | 104.39 | 104.89 | 96.40 |
| | | M3 | 104.96 | 104.53 | 105.10 | 96.56 |
| | | M_{avg} | 105.2 | 104.3 | 104.9 | 96.5 |
| Rosuvastatin Dissolution | Rosuvastatin Q = 75.0% in 30 minutes | M1 | 97.46 | 97.62 | 96.31 | 91.25 |
| | | M2 | 104.73% | 99.91% | 95.69% | 97.86% |
| | | M3 | 96.45% | 98.38% | 101.26% | 94.60% |
| | | M4 | 98.31 | 95.24 | 101.75% | 93.96% |
| | | M5 | 101.13% | 99.46% | 86.86% | 92.59% |
| | | M6 | 99.74% | 97.23% | 98.65 | 97.20% |
| | | M_{avg} | 99.6 | 98.0% | 98.4 | 94.6% |
| | | CV | 3.01 | 1.73 | 2.63 | 2.72 |
| Acetylsalicylic Acid Dissolution | Acetylsalicylic acid Q = 70.0% in 45 minutes | M1 | 84.05 | 82.37 | 80.54 | 95.15 |
| | | M2 | 86.60 | 83.01 | 83.42% | 100.32% |
| | | M3 | 90.98% | 81.90% | 89.24% | 93.23% |
| | | M4 | 93.02 | 84.35 | 80.05% | 93.78% |
| | | M5 | 95.61% | 83.71% | 90.77% | 95.76% |
| | | M6 | 82.89% | 88.44 | 92.49% | 90.51% |
| | | M_{avg} | 88.9 | 84.0 | 86.1 | 94.8% |
| | | CV | 5.77 | 2.81 | 6.30 | 3.45 |
| Organic impurities in Rosuvastatin | Rosuvastatin ketone: No more than 2.10% | M1 | 0.16 | 0.22 | 0.27 | 0.43 |
| | Rosuvastatin lactone: No more than 3.80% | M1 | 0.10 | 0.24 | 0.53 | 2.68 |
| | Rosuvastatin ethyl ester: No more than 0.50% | M1 | Not detected | Not detected | Not detected | Not detected |
| | Any non-specific degradation product: No more than 0.20% | M1 | 0.03 | 0.03 | 0.01 | 0.03 |
| | Total degradation products: No more than 3.60% | M1 | 0.08 | 0.07 | 0.06 | 0.15 |
| Salicylic acid limit | No more than 2.00% | X20 | 0.28 | 0.39 | 0.48 | 1.53 |
| Microbial limits | Aerobic mesophilic count: No more than 10³ CFU/g | X20 | Less than 10 CFU/g | Not applicable | Less than 10 CFU/g | Less than 10 CFU/g |
| | Filamentous fungi and yeasts: No more than 10² CFU/g | | Less than 10 CFU/g | Not applicable | Less than 10 CFU/g | Less than 10 CFU/g |
| | Absence of pathogens: Absence of *Escherichia coli* | | Absence of *Escherichia coli* | Not applicable | Absence of *Escherichia coli* | Absence of *Escherichia coli* |
| Water content | Tablet: No more than 15.00% | X20 | 4.64 | 4.64 | 5.15 | 4.72 |
| | Granules: No more than 15.00% | | 3.40 | 4.04 | 4.29 | 2.66 |

As to the product description, the tested batches met the specification in all analysis periods, i.e., the product description corresponded to a size #0 capsule with opaque white body and opaque red cap containing a white colored, biconvex, coated tablet plain on both sides, and white colored granules free of foreign particles.

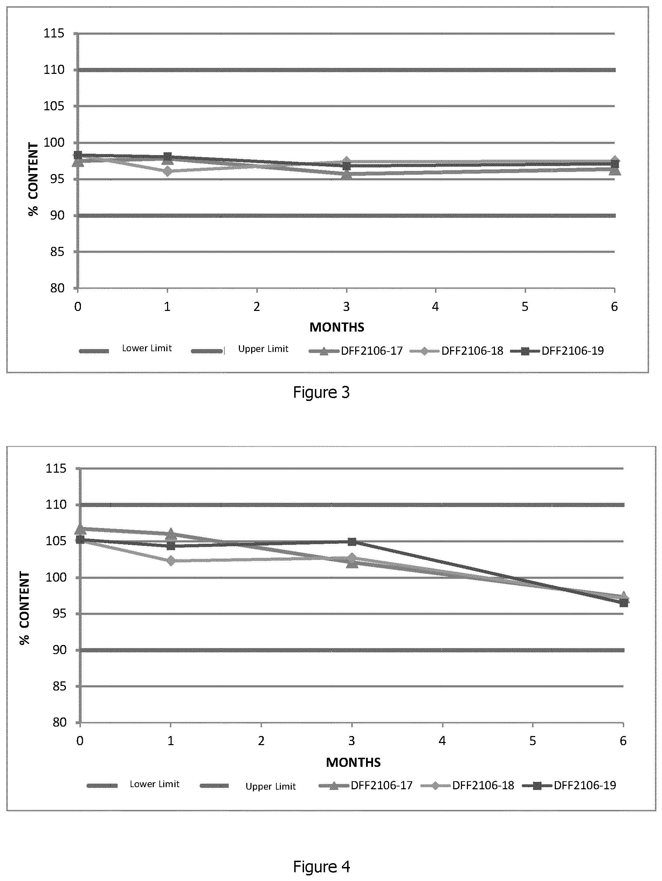

Figure 3 depicts a plot showing the results of the rosuvastatin content for the three tested batches. It can be seen that the rosuvastatin content underwent slight changes throughout the study, but the trend generally is that the content remained within the specification.

Similarly, Figure 4 is a plot showing the results of the acetylsalicylic acid content for the three tested batches. It can be seen that the acetylsalicylic acid content underwent slight changes throughout the study, but the trend generally is that the content remained within the specification.

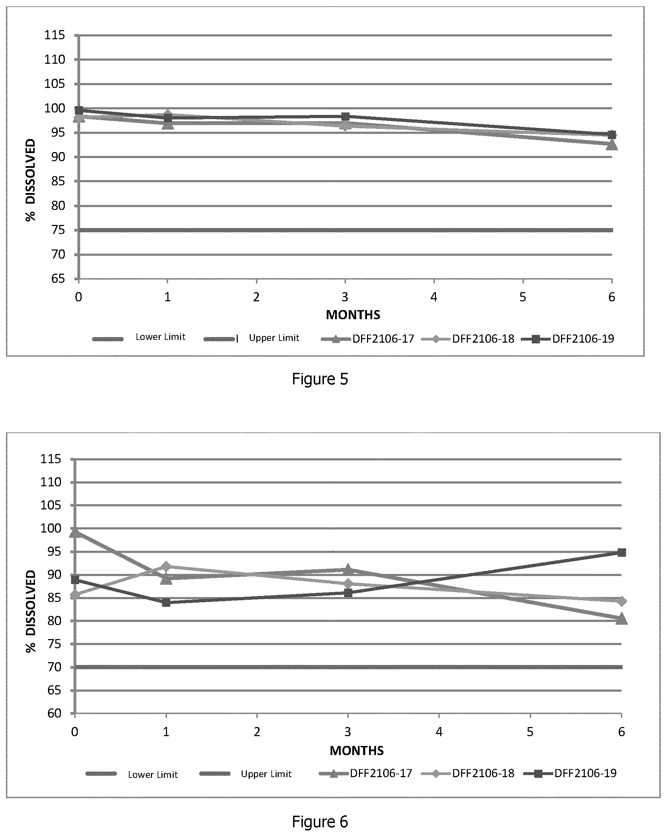

On the other hand, Figure 5 shows the results of rosuvastatin dissolution. It can be seen that the rosuvastatin dissolution decreased slightly for the three tested batches at the end of the study; however, despite such decrease, the percentage of dissolved rosuvastatin remains above the specification.

As to the acetylsalicylic acid dissolution (Figure 6), there was greater variation in results between batches; however, the percentage of acetylsalicylic acid was above 70.0% in the tested periods and in all batches, thus meeting the specification.

From the results set forth and described above, it can be concluded that the tested batches (DFF2106-17, DFF2106-18, and DFF2106-19) demonstrated that they retained their chemical and physical characteristics within the established specifications, thus ensuring their quality, efficacy, and safety.

### Example 3. Comparative bioavailability study

A comparative bioavailability study was conducted in human subjects to assess differences in the rate and extent of absorption between a pharmaceutical composition according to a preferred embodiment of the present invention and concomitant administration of the reference medicaments. The secondary aim of the study was to assess the safety and tolerability of a composition according to a preferred embodiment of the present invention in healthy male and female subjects under fasting conditions.

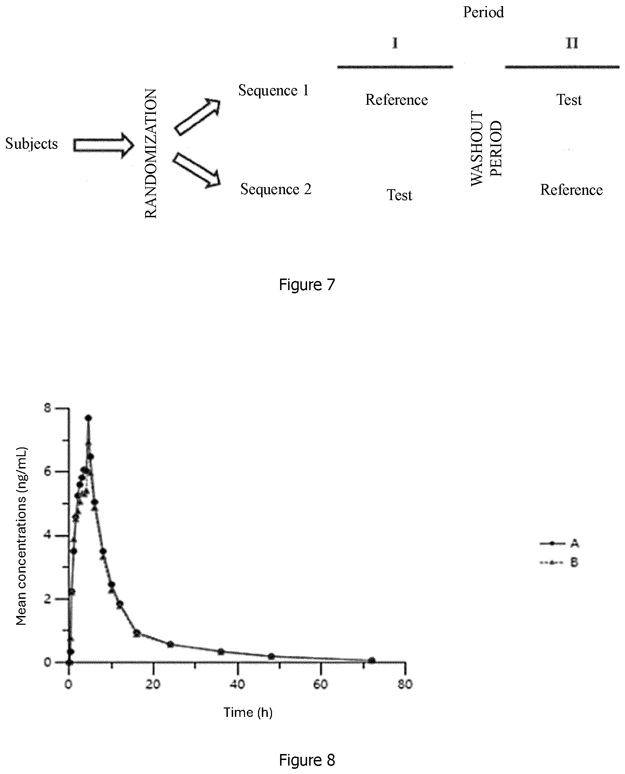

To this end, as illustrated in Figure 7, a single-dose, two-sequence, two-period, two-treatment, randomized, crossover, open-label, prospective, longitudinal, single-center design study was conducted to compare one capsule of the pharmaceutical composition according to a preferred embodiment of the present invention (test medicament) and one tablet of both reference medicaments (according to the randomization sequence), under fasting conditions, with 250 mL of water and a 7-day washout period between each dosing of the research product.

The reference medicaments were as follows:
- Crestor^{®}: Tablet comprising 10 mg rosuvastatin from AstraZeneca, S.A. de C.V.
- Aspirin Junior^{®}: Tablet comprising 100 mg acetylsalicylic acid from Bayer de México, S.A. de C.V.

As stated above, the test medicament corresponds to a pharmaceutical composition according to one embodiment of the present invention, particularly a pharmaceutical composition as described in Example 1 and Table 1.

The clinical trial was evaluated and approved by the Research Ethics Committee and the Research Committee of Avant Santé Research Center, S.A. de C.V. It was subsequently evaluated and authorized by the Federal Commission for Protection against Health Risks (COFEPRIS) under file number 213301410B0347/2021.

Prior to any process related to the clinical trial, the research subjects signed the Informed Consent Form after having received complete written and verbal information about the aspects involved in the trial, thus confirming their voluntary participation.

The trial consisted of two periods of hospitalization. In each period, the volunteers remained in the clinical trial unit for approximately 36 hours (12 hours prior to and 24 hours after dosing with the research products) in order to standardize the subjects' diet and assess the safety of the products. The trial periods were separated by a 7-day washout period.

Twenty-six whole blood samples (8 mL) were each taken (in each period) at the following timepoints: 0.0, 00.167, 00.25, 00.34, 00.50, 00.75, 01.00, 01.25, 01.50, 01.75, 02.00, 02.50, 03.00, 03.50, 04.00, 04.50, 05.00, 06.00, 08.00, 10.00, 12.00, 16.00, 24.00, 36.00, 48.00, and 72.00 hours after dosing with the research products. Samples were collected in Vacutainer 8 mL tubes containing K₂EDTA (dipotassium ethylenediaminetetraacetic acid) as an anticoagulant. There was a 2-minute time lag between each of the trial groups. Rosuvastatin and salicylic acid (a metabolite of acetylsalicylic acid) were quantified by reversed-phase ultrahigh-performance liquid chromatography coupled to a mass-to-mass detector.

### Population

Thirty-six research subjects were included in the clinical trial, of whom 21 were men and 15 were women aged between 19 and 50 years, with an average age of 29 years.

During the first period of the trial, subject 03 was removed from the trial due to an adverse event (COVID-19), which was not related to the treatment. During the second period of the trial, subjects 32 and 33 were removed from the trial due to withdrawal of informed consent. According to the aforementioned, of 36 research subjects, 33 completed their participation in the clinical trial.

### Treatment administered

Table 6 shows the characteristics of the treatments administered.

**Table 6. Treatments administered, description of medicaments**

| **Medicament details** | **Test medicament (A)** | **Reference medicament (B₁)** | **Reference medicament (B₂)** |
|---|---|---|---|
| International nonproprietary name | Rosuvastatin/ Acetylsalicylic acid | Rosuvastatin | Acetylsalicylic acid |
| Distinctive name | N/A | CRESTOR^{®} | ASPIRINA JUNIOR^{®} |
| Pharmaceutical form | Capsules | Tablets | Tablets |
| Dosage | 10 mg/100 mg | 10 mg | 100 mg |
| Concentration | 10 mg/100 mg | 10 mg | 100 mg |
| Doses administered | 35 | 34 | 34 |

### Vital signs

Vital signs, including blood pressure, respiratory rate, heart rate, and temperature, were taken upon admission, prior to dose administration, at 01.00 and 03.00 hours (within a range of ±45 minutes) after the dose, and prior to discharge in each trial period.

### Adverse events

During the trial, a total of 4 adverse events occurred, of which 2 were "headache", 1 was "COVID-19", and 1 was "decreased systolic blood pressure". Based on the severity of the event, 3 events were classified as "mild" and 1 as "moderate". Based on causality, 2 events were classified as "likely", 1 as "possible", and 1 as "unlikely". Regarding the safety assessment, 2 events were considered "expected" and 2 "unexpected". Under the provisions set forth in the authorized clinical protocol and NOM-220-SSA1-2016 "Installation and operation of the pharmacovigilance" in force, all adverse events were classified as non-serious and unrelated to treatment. Table 7 describes the adverse events that occurred.

**Table 7. Description of adverse events**

| Subject Number | Treatment administered | Patient gender | Patient age (years) | Severity of report | Adverse event reported | Severity | Causality | Expected or unexpected event |
|---|---|---|---|---|---|---|---|---|
| 26 | Reference medicament (B₁ and B₂) | Male | 24 | Not serious | Headache | Moderate | Possible | Expected |
| 03 | Reference medicament (B₁ and B₂) | Male | 22 | Not serious | COVID-19 | Mild | Unlikely | Unexpected |
| 05 | Reference medicament (B₁ and B₂) | Male | 24 | Not serious | Decreased systolic blood pressure | Mild | Possible | Unexpected |
| 17 | Reference medicament (B₁ and B₂) | Male | 50 | Not serious | Headache | Mild | Possible | Expected |

### Pharmacokinetic bioavailability between treatments

To determine that the test medicament (A) has similar comparative bioavailability to concomitant administration of reference medicaments B₁ and B₂, the confidence intervals for the geometric means of the ratio (test/reference) of maximum plasma concentration parameters (Cₘₐₓ) and ABC of Rosuvastatin and salicylic acid (metabolite of acetylsalicylic acid) must be between 80% and 125%, and the two one-sided t-tests of Schuirmann must show a value less than 0.05.

The comparative bioavailability results are shown in Tables 8 and 9.

**Table 8. Pharmacokinetic parameters of the medicaments administered**

| Parameter | A | | B₁ + B₂ | |
|---|---|---|---|---|
| | Rosuvastatin | Salicylic acid | Rosuvastatin | Salicylic acid |
| Cₘₐₓ (ng/mL) | 7.892 ± 4.6560 | 7359.168 ± 1485.6889 | 7.162 ± 4.2202 | 7352.475 ± 1669.0617 |
| AUC₀₋ₜ (h*ng/mL) | 72.467 ± 40.0741 | 28939.434 ± 7336.1749 | 68.085 ± 40.9434 | 28981.949 ± 8143.0935 |
| AUC_{0-inf} (h*ng/mL) | 75.484 ± 40.1511 | 30250.490 ± 7247.0982 | 71.446 ± 40.7350 | 30295.059 ± 8295.9829 |
| t₍ₘₐₓ) (h) | 4.288 ± 0.9273 | 1.508 ± 0.5247 | 3.924 ± 1.2382 | 1.833 ± 0.8585 |
| Kel (1/h) | 0.047 ± 0.0150 | 0.379 ± 0.0802 | 0.048 ± 0.0221 | 0.376 ± 0.0719 |
| t_{1/2} (h) | 16.585 ± 6.6856 | 1.907 ± 0.3915 | 17.376 ± 9.0628 | 1.922 ± 0.4409 |

**Table 9. Results of statistical bioavailability tests between medicaments with data transformed into their natural logarithm.**

| Parameter | | A/B (%) | Geometric mean of least squares | | Confidence interval | Schuirmann | | Statistical power (%) |
|---|---|---|---|---|---|---|---|---|
| | | | A | B | (80.00 - 125.00%) | P<80.00 | P>125.00 | |
| Rosuvastatin | Cₘₐₓ | 109.7 | 6,848 | 6,244 | 98.28 - 122.39 | 0.0000 | 0.0260 | 95.5 |
| | AUC₀₋₄ | 107.0 | 63.672 | 59.495 | 97.56 - 117.39 | 0.0000 | 0.0039 | 98.9 |
| Salicylic acid | Cₘₐₓ | 100.7 | 7198.863 | 7146.769 | 93.97 - 107.97 | 0.0000 | 0.0000 | 100.0 |
| | AUC₀₋₄ | 100.4 | 28002.313 | 27902.361 | 95.95 - 104.97 | 0.0000 | 0.0000 | 100.0 |

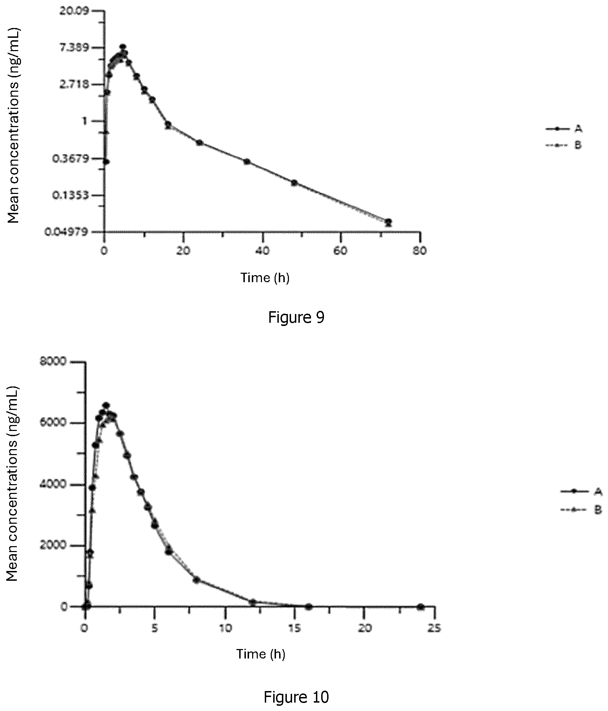

Figures 8 and 9 show the pharmacokinetic profile of the maximum concentration of treatment A (pharmaceutical composition according to one embodiment of the present invention) and concomitant administration of reference medicaments B₁ and B₂.

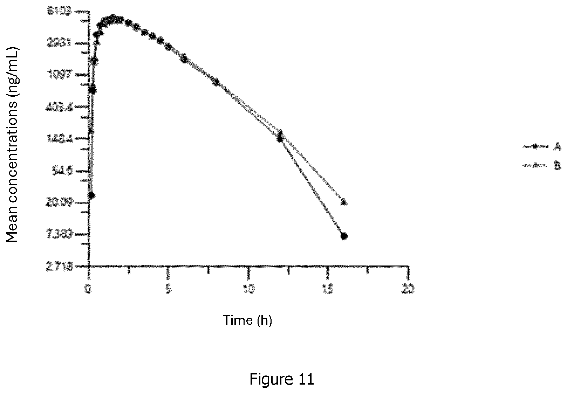

Figures 10 and 11 depict the pharmacokinetic profile of the maximum concentration of treatment A (pharmaceutical composition according to one embodiment of the present invention) and concomitant administration of reference medicaments B₁ and B₂ on a normal and semi-logarithmic scale for salicylic acid.

Statistical analysis between test medicament A (pharmaceutical composition according to one embodiment of the present invention) and reference medicaments B₁ and B₂ showed that the pharmacokinetic parameters, maximum plasma concentration (Cₘₐₓ) and area under the plasma concentration curve from time zero to the last measurable concentration (AUC₀₋ₜ), are directly related to each other and without statistically significant differences in the rate of absorption and the amount of drug absorbed, and the confidence intervals of the pharmacokinetic parameters described above are within the ranges of 80%-125% for transformed data of Rosuvastatin/Salicylic Acid. Accordingly, the bioavailability of test product A (pharmaceutical composition according to one embodiment of the present invention) is comparable to reference medicaments B₁ and B₂.

According to the matter described above, it can be seen that the pharmaceutical composition comprising rosuvastatin and acetylsalicylic acid has been conceived to provide a stable, immediate-release pharmaceutical form with a bioavailability of the active ingredients similar to that of the co-administered monodrugs, and it will be evident to any person skilled in the art that embodiments with regard to a pharmaceutical composition comprising rosuvastatin and acetylsalicylic acid, process for preparing the same, and uses thereof as described above and illustrated in the accompanying drawings are only illustrative and not limiting of the present invention, since numerous changes of consideration are possible in their details without departing from the scope of the invention.

Therefore, the present invention shall not be considered as restricted except for what is required by the prior art and the scope of the attached claims.

## Claims

1. A pharmaceutical composition, **characterized by** comprising: a) a first phase comprising rosuvastatin, and b) a second phase comprising acetylsalicylic acid,
wherein the first phase comprising rosuvastatin is in the form of a coated tablet, and the second phase comprising acetylsalicylic acid is in the form of granules.

2. The pharmaceutical composition according to claim 1, further comprising pharmaceutically acceptable excipients selected from the group consisting of diluents, carriers, disintegrants, glidants, lubricants, coatings, binders, wetting agents, and stabilizing agents.

3. The pharmaceutical composition according to claim 1, wherein the first phase comprising rosuvastatin further comprises:
at least one diluent selected from the group consisting of lactose monohydrate, microcrystalline cellulose, anhydrous calcium phosphate, and corn starches;
a stabilizing agent selected from the group consisting of phosphate derivatives including anhydrous dibasic calcium phosphate, and aluminum magnesium silicate;
a disintegrant selected from the group consisting of croscarmellose sodium, cellulose derivatives including hydroxypropyl celluloses, carboxymethyl celluloses, microcrystalline cellulose; povidone derivatives including plasdones, starch derivatives including pregelatinized starch, sodium glycolate starch, and corn starch;
a glidant selected from the group consisting of colloidal silicon dioxide; cellulose derivatives including carboxymethyl celluloses, microcrystalline cellulose, starch derivatives including pregelatinized starch and corn starch, silicate derivatives including magnesium silicate, magnesium trisilicate, and talc; and
a lubricant selected from the group consisting of stearate derivatives including zinc stearate, magnesium stearate, calcium stearate, stearic acid, monostearates, stearyl fumarate, sulfated derivatives including magnesium lauryl sulfate, and talc.

4. The pharmaceutical composition according to claim 3, wherein the first phase comprising rosuvastatin comprises two lubricants.

5. The pharmaceutical composition according to claim 3 or 4, wherein the first phase comprising rosuvastatin further comprises lactose monohydrate as a diluent, dibasic calcium phosphate as a stabilizing agent, croscarmellose sodium as a disintegrant, colloidal silicon dioxide as a glidant, talc as a first lubricant, and zinc stearate as a second lubricant, and a polyvinyl acetate coating.

6. The pharmaceutical composition according to any of claims 3 to 5, wherein the first phase comprising rosuvastatin further comprises:
- rosuvastatin or a pharmaceutically acceptable salt thereof as an active ingredient in a dose of from 10 mg to 20 mg rosuvastatin;
- lactose monohydrate in an amount of from 40% to 80% by weight of the tablet;
- dibasic calcium phosphate as a stabilizing agent in an amount of from 0.26% to 2% by weight of the tablet;
- sodium croscarmellose as a disintegrant in an amount of from 0.5% to 15% by weight of the tablet;
- colloidal silicon dioxide as a glidant in an amount of from 0.08% to 2% by weight of the tablet;
- talc as a first lubricant in an amount of from 0.5% to 10% by weight of the tablet;
- zinc stearate as a second lubricant in an amount of from 0.5% to 10% by weight of the tablet; and
- a polyvinyl acetate coating in an amount of from 0.5% to 10% by weight of the tablet.

7. The pharmaceutical composition according to claim 6, wherein the rosuvastatin is rosuvastatin calcium.

8. The pharmaceutical composition according to claim 7, wherein the tablet comprises from 10.40 mg to 20.80 mg rosuvastatin calcium.

9. The pharmaceutical composition according to any of claims 4 to 8, wherein the coating of the rosuvastatin tablet is in an amount of from 0.5% to 6% by weight of the tablet.

10. The pharmaceutical composition according to claim 1, wherein the second phase comprising acetylsalicylic acid further comprises:
at least one of a diluent selected from the group consisting of starch derivatives including pregelatinized starch and corn starch; cellulose derivatives including microcrystalline cellulose, carbomers, hydroxypropyl cellulose, and carboxymethyl cellulose; polyethylene glycol in its different grades; and poloxamer;
a binder selected from the group consisting of glyceryl dibehenate; povidones and derivatives thereof; cellulose derivatives including carbomers, hydroxypropyl cellulose, carboxymethyl cellulose; starch derivatives including pregelatinized starch and corn starch; polyethylene glycol in its different grades; and poloxamer;
a wetting agent selected from the group consisting of sodium lauryl sulfate; poloxamers; polyoxyethylene castor oil derivative; benzalkonium chloride; benzetonium chloride; polyoxyethylene alkyl ethers; and sorbitan polyoxyethylene fatty acid esters;
a glidant selected from the group consisting of colloidal silicon dioxide; cellulose derivatives including carboxymethyl cellulose and microcrystalline cellulose; starch derivatives including pregelatinized starch and corn starch; silicate derivatives including magnesium silicate and magnesium trisilicate; and talc; and
a lubricant selected from the group consisting of stearate derivatives including magnesium stearate, zinc stearate, calcium stearate, stearic acid, monostearates, and stearyl fumarate; sulfated derivatives including magnesium lauryl sulfate and sodium lauryl sulfate; and talc.

11. The pharmaceutical composition according to claim 10, wherein the binder is glyceryl dibehenate, the wetting agent is lauryl sulfate, the glidant is colloidal silicon dioxide, the lubricant is zinc stearate, and the diluent is selected from the group consisting of pregelatinized starch and microcrystalline cellulose.

12. The pharmaceutical composition according to claim 10 or 11, wherein the acetylsalicylic acid granules comprise two diluents.

13. The pharmaceutical composition according to any of claims 10 to 12, wherein the acetylsalicylic acid granules comprise:
- acetylsalicylic acid as an active ingredient in a dose of 100 mg;
- pregelatinized starch as a first diluent in an amount of from 5% to 75% by weight of the granules;
- glyceryl dibehenate as a binder in an amount of from 0.4% to 4% by weight of the granules;
- microcrystalline cellulose as a second diluent in an amount of from 5% to 90% by weight of the granules;
- sodium lauryl sulfate as a wetting agent in an amount of from 0.02% to 4% by weight of the granules;
- colloidal silicon dioxide as a glidant in an amount of from 0.08% to 2% by weight of the granules; and
- zinc stearate as a lubricant in an amount of from 0.1% to 1%.

14. The pharmaceutical composition according to any of claims 1 to 13, wherein it is a solid form.

15. The pharmaceutical composition according to any of claims 1 to 14, wherein it is a capsule.

16. The pharmaceutical composition according to any of claims 1 to 15, wherein it comprises:
(a) a first phase comprising:
- 10.4 mg rosuvastatin calcium;
- 50.20 mg lactose monohydrate;
- 1.20 mg dibasic calcium phosphate;
- 3.5 mg sodium croscarmellose;
- 0.40 mg colloidal silicon dioxide;
- 3.6 mg talc;
- 0.70 mg zinc stearate; and
- 3 mg of a polyvinyl acetate coating;
wherein the first rosuvastatin phase is in the form of a coated tablet;
(b) a second phase comprising:
- 100 mg acetylsalicylic acid;
- 129.10 mg pregelatinized starch;
- 10 mg glyceryl dibehenate;
- 50 mg microcrystalline cellulose;
- 9.4 mg sodium lauryl sulfate;
- 0.75 mg colloidal silicon dioxide; and
- 0.75 mg zinc stearate;
wherein the second acetylsalicylic acid phase is in the form of granules; and
the pharmaceutical form containing the first rosuvastatin phase and the second acetylsalicylic acid phase is a capsule.

17. The pharmaceutical composition according to any of claims 1 to 15, wherein it comprises:
(a) a first phase comprising:
- 20.8 mg rosuvastatin calcium;
- 39.80 mg lactose monohydrate;
- 1.20 mg dibasic calcium phosphate;
- 3.5 mg sodium croscarmellose;
- 0.40 mg colloidal silicon dioxide;
- 3.6 mg talc;
- 0.70 mg zinc stearate; and
- 3 mg of a polyvinyl acetate coating;
wherein the first rosuvastatin phase is in the form of a coated tablet;
(b) a second phase comprising:
- 100 mg acetylsalicylic acid;
- 129.10 mg pregelatinized starch;
- 10 mg glyceryl dibehenate;
- 50 mg microcrystalline cellulose;
- 9.4 mg sodium lauryl sulfate;
- 0.75 mg colloidal silicon dioxide; and
- 0.75 mg zinc stearate;
wherein the second acetylsalicylic acid phase is in the form of granules; and
the pharmaceutical form containing the first rosuvastatin phase and the second acetylsalicylic acid phase is a capsule.

18. A process for manufacturing a pharmaceutical composition as described in any of claims 1 to 17, **characterized in that** it comprises the following stages:
a) providing a first phase comprising rosuvastatin, wherein the first phase comprising rosuvastatin is in the form of a coated tablet;
b) providing a second phase comprising acetylsalicylic acid, wherein the second phase comprising acetylsalicylic acid is in the form of granules; and
c) encapsulating the first phase comprising rosuvastatin and the second phase comprising acetylsalicylic acid.

19. The process according to claim 18, wherein the first phase comprising coated rosuvastatin is prepared by the following steps:
- mixing the rosuvastatin with a glidant, a disintegrant, a stabilizing agent, a diluent, and a first lubricant;
- lubricating the above mixture with at least one lubricant;
- compressing the lubricated mixture to obtain a rosuvastatin tablet;
- coating the tablet.

20. The process according to claim 19, wherein the diluent is lactose monohydrate, the stabilizing agent is dibasic calcium phosphate, the disintegrant is croscarmellose sodium, the glidant is colloidal silicon dioxide, the solvent is purified water, the first lubricant is talc, the second lubricant is zinc stearate, and the coating is polyvinyl acetate.

21. The process according to claim 18, wherein the second phase comprising acetylsalicylic acid is prepared by the following steps:
- mixing the acetylsalicylic acid with a wetting agent, a first diluent, and a binder;
- granulating and heating the above mixture;
- cooling the mixture and adding a glidant and a second diluent;
- lubricating the mixture with a lubricant.

22. The process according to claim 19, wherein the binder is glyceryl dibehenate, the wetting agent is lauryl sulfate, the glidant is colloidal silicon dioxide, the lubricant is zinc stearate, the first diluent is pregelatinized starch, and the second diluent is microcrystalline cellulose.

23. The process according to claim 18, wherein the encapsulating stage c) comprises put the first phase comprising rosuvastatin and the second phase comprising acetylsalicylic acid into a gelatin capsule.

24. A composition as claimed in any of claims 1 to 17 for use in the treatment of hyperlipidemia or prevention of at least one cardiovascular disease.

25. The composition for use according to claim 24, wherein hyperlipidemia and cardiovascular disease are selected from the group consisting of hyperlipidemia, hyperlipoproteinemia, hypercholesterolemia, hypertension, angina pectoris, heart attacks, and aneurysms.

26. The composition for use according to claim 25, wherein the pharmaceutical composition regulates the blood concentration of low-density lipoproteins (LDL), high-density lipoproteins (HDL), triglycerides, and cholesterol.
